# EUROPÄISCHE PATENTSCHRIFT

(11) **EP 2 092 087 B1**
(45) Veröffentlichungstag und Bekanntmachung des Hinweises auf die Patenterteilung: **09.07.2014**
(21) Anmeldenummer: 07870196.8
(22) Anmeldetag: 01.11.2007
(51) Int. Cl.: C12Q 1/68

(54) **PROGNOSTISCHE MARKER FÜR DIE KLASSIFIZIERUNG VON KOLOREKTALEN KARZINOMEN BASIEREND AUF EXPRESSIONSPROFILEN VON BIOLOGISCHEN PROBEN**
PROGNOSTIC MARKERS FOR CLASSIFYING COLORECTAL CARCINOMA ON THE BASIS OF EXPRESSION PROFILES OF BIOLOGICAL SAMPLES
MARQUEURS DE PRONOSTIC POUR LA CLASSIFICATION DE CARCINOMES COLORECTAUX SUR LA BASE DE PROFILS D'EXPRESSION D'ÉCHANTILLONS BIOLOGIQUES

(30) Priorität: 02.11.2006 DE 102006035388
(43) Veröffentlichungstag der Anmeldung: 26.08.2009
(73) Patentinhaber: Signature Diagnostics AG, 14473 Potsdam (DE)
(72) Erfinder: HINZMANN, Bernd, 13127 Berlin (DE); ADAMS, Hans-Peter, 14469 Potsdam (DE); MAYR, Tobias, 10435 Berlin (DE); CLEVERT, Djörk-Arné, 10437 Berlin (DE)
(74) Vertreter: Hoppe, Georg Johannes
(86) Internationale Anmeldenummer: PCT/DE2007/050005
(87) Internationale Veröffentlichungsnummer: WO 2008/061527

(56) Entgegenhaltungen:
- STOSS O ET AL: "RNA expression profiling in cardiac tissue - a successful route to new drug targets?" TARGETS, ELSEVIER, Bd. 1, Nr. 1, 1. Juli 2002 (2002-07-01), Seiten 12-19, XP004886582 ISSN: 1477-3627
- ARANGO ET AL: "Gene-Expression Profiling Predicts Recurrence in Dukes' C Colorectal Cancer" GASTROENTEROLOGY, ELSEVIER, PHILADELPHIA, PA, Bd. 129, Nr. 3, 1. September 2005 (2005-09-01), Seiten 874-884, XP005314702 ISSN: 0016-5085
- WANG Y ET AL: "Gene expression profiles and molecular markers to predict recurrence of Dukes' B colon cancer" JOURNAL OF CLINICAL ONCOLOGY, GRUNE AND STRATTON, NEW YORK, NY, Bd. 22, Nr. 9, 1. Mai 2004 (2004-05-01), Seiten 1564-1571, XP003014769 ISSN: 0732-183X
- ESCHRICH S ET AL: "Molecular staging for survival prediction of colorectal cancer patients" JOURNAL OF CLINICAL ONCOLOGY, GRUNE AND STRATTON, NEW YORK, NY, Bd. 23, Nr. 15, 20. Mai 2005 (2005-05-20), Seiten 3526-3535, XP003014768 ISSN: 0732-183X
- BERTUCCI FRANCOIS ET AL: "Gene expression profiling of colon cancer by DNA microarrays and correlation with histoclinical parameters" ONCOGENE, BASINGSTOKE, HANTS, GB, Bd. 23, Nr. 7, 19. Februar 2004 (2004-02-19), Seiten 1377-1391, XP002414422 ISSN: 0950-9232
- GAMBERONI GIACOMO ET AL: "Finding biological process modifications in cancer tissues by mining gene expression correlations" BMC BIOINFORMATICS, BIOMED CENTRAL, LONDON, GB, Bd. 7, Nr. 1, 9. Januar 2006 (2006-01-09), Seite 6, XP021001116 ISSN: 1471-2105
- YULAN LIANG ET AL: "Associating phenotypes with molecular events: recent statistical advances and challenges underpinning microarray experiments" FUNCTIONAL & INTEGRATIVE GENOMICS, SPRINGER-VERLAG, BE, Bd. 6, Nr. 1, 1. Januar 2006 (2006-01-01), Seiten 1-13, XP019356420 ISSN: 1438-7948
- JAFARI PEYMAN ET AL: "An assessment of recently published gene expression data analyses: reporting experimental design and statistical factors" BMC MEDICAL INFORMATICS AND DECISION MAKING, BIOMED CENTRAL, LONDON, GB, Bd. 6, Nr. 1, 21. Juni 2006 (2006-06-21), Seite 27, XP021017521 ISSN: 1472-6947
- 'Medline Search for Gene expression analysis' 02 November 2006, XP055062398

## Beschreibung

### Zusammenfassung der Erfindung

Die Erfindung umfasst ein Verfahren zur Vorhersage (Prädiktion) der Progression einer Dickdarmkrebserkrankung (kolorektales Karzinom) innerhalb von drei Jahren bei Patienten, die mit Dickdarmkrebs im UICC Stadium I und II nach dem Stand der Technik diagnostiziert wurden und deren primärer Tumor nach chirurgischen und pathologischen Kriterien vollständig (R0) entfernt (reseziert) wurde. Das erfindungsgemäße Verfahren beinhaltet die Bestimmung und Analyse des Expressions profiles der Markergene ME2 und FAS in einer Gewebeprobe des bei der Operation entfernten primären Tumors des Patienten. Mit Hilfe des Verfahrens wird für den einzelnen Patienten vorhergesagt, ob er/sie innerhalb von drei Jahren nach der Operation mit einer Progression der Krebserkrankung rechnen muss oder nicht. Als Progression der Erkrankung gilt hier die fachgerechte medizinische Feststellung (Diagnose) eines Rezidivs der Erkrankung im selben Organ, einer Tochtergeschwulst (Metastase) in anderen Organen, oder das Auftreten anderer Krebserkrankungen. Das Verfahren erlaubt, anders ausgedrückt, die Vorhersage des dreijährigen progressionsfreien Überlebens von Dickdarmkrebspatienten durch Bestimmung eines Genexpressionsprofils von ME2 und FAS sowie der sich anschließenden bioinformatischen Auswertung. Die Gene sind durch ihre Sequenzen wie dargestellt in den SEQ ID NOs: 1 und 2 definiert. Ebenfalls offenbart werden Kits zur Durchführung des erfinderischen Verfahrens.

### Hintergrund der Erfindung und Stand der Technik

Dickdarmkrebs, auch als kolorektales Karzinom bezeichnet, stellt die dritthäufigste Tumorentität in westlichen Ländern dar. In Deutschland erkranken jedes Jahr etwa 66.000 Patienten an Dickdarmkrebs. Das kolorektale Karzinom ist eine heterogene Krankheit mit komplexer Ursache. Dickdarmkrebspatienten werden aufgrund von histopathologischen Kriterien, die von der Union International Contre le Cancer (UICC) definiert wurden, in vier klinische Stadien, UICC I-IV, eingeteilt. Das TNM-Klassifikationschema der UICC wird weltweit verwendet.

Patienten mit Dickdarmkrebs im UICC Stadium I haben einen TNM-Status von T_{1/2}N₀M₀. Bei diesen Patienten sind keine regionalen Lymphknoten mit Metastasen befallen (N=0) und es sind auch keine Metastasen festgestellt und histologisch gesichert worden (M=0).

Patienten mit Dickdarmkrebs im Stadium II haben einen TNM-Status von T_{3.4}N₀M₀. Obwohl der Primärtumor deutlich größer ist als im Stadium I und auch bereits die Darmwand durchdrungen hat, sind bei diesen Patienten keine Metastasen in den regionalen Lymphknoten und keine Metastasen festgestellt worden.

Etwa die Hälfte aller neu-diagnostizierten Patienten, in Deutschland etwa 33.000 Patienten pro Jahr, haben Dickdarmkrebs im UICC Stadium I und II. Die chirurgische Komplettresektion von Tumoren in den klinischen Stadien I und II ist sehr effektiv und führt zu progressionsfreien Überlebensraten von 76% nach 5 Jahren im UICC Stadium I und 67% im UICC Stadium II. Jedoch kommt es innerhalb von 5 Jahren nach der operativen Komplettentfernung, des Primärtumors bei etwa 24% der Dickdarmkrebspatienten im UICC Stadium I und bei 33% der Dickdarmkrebspatienten im UICC Stadium II zu einem Fortschreiten (Progression) der Krebserkrankung. Die Diagnose von Tochtergeschwülsten (Metastasen) des Primärtumors in Leber und/oder Lunge konstituieren die Mehrzahl der beobachteten Progressionen.

Patienten im UICC Stadium III haben einen TNM-Status T₁₋₄, N₁₋₂M₀. Für Patienten in diesem ist typisch, dass regionale Lymphknoten bereits mit Absiedelungen des Primärtumors befallen sind, jedoch noch keine Metastasen in anderen Organen festgestellt wurden. Das Vorhandensein von befallenen Lymphknoten im UICC Stadium III erhöht die Wahrscheinlichkeit der Progression der Erkrankung beträchtlich. Etwa 60% der Patienten im Stadium III müssen mit Progressionen der Erkrankung innerhalb von fünf Jahren nach der chirurgischen Entfernung des Primärtumors rechnen. Aufgrund dieser hohen Progressionsrate bekommen Patienten im UICC Stadium III nach den Richtlinien der deutschen Krebsgesellschaft eine unterstützende (adjuvante) Chemotherapie. Die adjuvante Chemotherapie verringert das Auftreten von Progressionen um etwa 10-20%, so dass in der Regel nur etwa 40-50% der Stadium III Patienten nach Operation und adjuvanter Chemotherapie innerhalb der ersten 5 Jahre eine Progression der Erkrankung erleiden.

Dickdarmkrebspatienten, bei denen bereits bei der Erstdiagnose Metastasen festgestellt und histologisch gesichert wurden, werden in das UICC Stadium IV eingeteilt. Sie haben nur eine relativ geringe 5-Jahresüberlebenswahrscheinlichkeit. In Deutschland betrifft dies etwa 20.000 Patienten. Bei diesen Patienten treten synchron oder metachron Lungen- oder Lebermetastasen auf. Bei etwa ca. 4.000 der Patienten im UICC Stadium IV sind eine Entfernung des primären Tumors und eine komplette Metastasenresektion (RO) technisch möglich, die mit einer 5-Jahres-Überlebensrate von ca. 30% einhergeht. Bei den übrigen 16.000 Patienten im UICC Stadium IV ist aus verschiedensten Gründen (multinodulär, ungünstige Lokalisation der Metastasen an Gefäßen und Gallengängen, extrahepatisch) eine Resektion nicht möglich. In diesen Fällen sind palliative Therapieoptionen indiziert. Ziel der palliativen chemotherapeutischen Behandlung ist neben der Aufrechterhaltung einer guten Lebensqualität die Verlängerung der Überlebenszeit.

Es gibt eine Reihe von Problemen bei der Klassifikation und Stadiumeinteilung der Dickdarmkrebspatienten. Die Einteilung von Patienten in das Stadium I und II ist nicht genau. Etwa 10% der Stadium I Patienten und etwa 25% der Stadium II Patienten erleiden innerhalb von 5 Jahren, die Mehrzahl bereits innerhalb von zwei Jahren nach operativer Entfernung des Primärtumors eine Progression. Allein in Deutschland betrifft dies etwa 6000-8000 Patienten jährlich. Es gibt keine Möglichkeit, die Patienten mit hoher Progressionswahrscheinlichkeit aus der homogen erscheinenden Gruppe herauszufinden. Seit längerer Zeit wird in Fachkreisen darüber diskutiert, ob man Patienten im UICC Stadium II grundsätzlich eine unterstützende (adjuvante) Chemotherapie verabreicht. Wegen der relativ geringen Progressionswahrscheinlichkeit von 33% innerhalb von 5 Jahren bei Stadium II Patienten, ist der Nutzen einer solchen Therapie im Voraus nur schwer zu bestimmen und bleibt deshalb kontrovers diskutiert. Ungefähr 67% aller Patienten im Stadium II würden von einer adjuvanten Chemotherapie nicht profitieren. Die Kosten wären enorm hoch.

Basierend auf prädiktiven Markern könnte eine individuelle Therapieentscheidung gefällt werden. In diesem Zusammenhang gab es viele Versuche, neue Marker zu finden, welche Patienten mit erhöhtem Progressionsrisiko identifizieren. Hawkins et al. (2002) Gastroenterology 122:1376-1387 untersuchten in diesem Zusammenhang Mikrosatelliteninstabilität und Promotermethylierung. Noura et al. (2002) J Clin Oncol 20:4232-benutzten einen RT-PCR basierten Nachweis von Lymphknotenmikrometastasen. Zhou et al. (2002) Lancet 359:219-225 untersuchten Allelungleichgewichte, um Rezidive in kolorektalen Karzinomen vorher sagen zu können. Eschrich et al. (2005) J Clin Oncol. 2005 May 20;23(15):3526-35 benutzten cDNA Microarrays, um die Überlebenswahrscheinlichkeit von Kolorektalkrebspatienten vorherzusagen.

Allen in der Literatur untersuchten Markern ist gemeinsam, dass sie bisher nicht als Grundlage prognostischer Assays in der klinischen Praxis angewendet werden, da sie nicht unabhängig validiert sind. Eine mögliche Ursache dafür könnte darin liegen, dass die Progression des kolorektalen Karzinoms eine Folge verschiedenster genetischer Ereignisse ist, welche innerhalb des malignen Epithels stattfinden, oder durch modifizierende Ereignisse des umgebenden stromalen Gewebes induziert werden. Um die potentielle Komplexität des Fortschreitens der Erkrankung verstehen zu können, bedarf es einer umfassenderen Analyse der zu Grunde liegenden molekularen Ereignisse.

Wang et al. ("Gene-Expression profiles and molecular markers to predict recurrence of Dukes' B colon cancer", JOURNAL OF CLINICAL ONCOLOGY, Bd. 22, Nr. 9, 1. Mai 2004, Seiten 1564-1571) beschreibt Gen-Expressionsprofile und molekulare Marker zur Prädiktion des Wiederauftretens von Dukes' B Dickdarmkrebs.

Eschrichs et al. ("Molecular staging for survival prediction of colorectal cancer patients", JOURNAL OF CLINICAL ONCOLOGY, Bd. 23, Nr. 15, 20. Mai 2005, Seiten 3526-3535) beschreibt die Vorhersage des Überlebens von Patienten mit Dickdarmkrebs.

Bertucci et al. ("Gene expression profiling of colon cancer by DANN microarrays and correlation with histoclinical parameters", ONCOGENE, Bd. 23, Nr. 7, 19. Februar 2004, Seiten 1377-1391) offenbart Gen-Expressionsprofile des Dickdarmkrebses mittels DNA Mikroarrays und deren Korrelation mit histoklinischen Parametern.

Gamberoni et al. ("Finding biological process modifications in cancer tissues by mining gene expression correlations", BMC BIONINFORMATICS, Bd. 7, Nr. 1, 9. Januar 2006, Seite 6) beschreibt biologische Prozessmodifikationen in Krebsgeweben.

Liang und Kelemen ("Associating phenotypes with molecular events: recent statistical advances and challenges underpinning microarray experiments", FUNCTIONAL & INTEGRATIVE GENOMICS, Bd. 6, Nr. 1, 1. Januar 2006, Seiten 1-13) beschreiben statistische Fortschritte und Herausforderungen hinsichtlich Mikroarray-Experimenten.

### Technisches Problem der Erfindung

Das der Erfindung zugrunde liegende technische Problem besteht in der Bereitstellung einer verlässlichen Diagnostik, die zu verbesserten individuellen Therapien führen kann.

Das technische Problem ist durch die Bereitstellung der hierin offenbarten Ausführungsformen und im Besonderen durch die die Erfindung charakterisierenden Ansprüche gelöst. Die Erfindung umfasst daher ein Verfahren zur Vorhersage der Wahrscheinlichkeit einer Progression (Lokalrezidiv, Metastasen, Zweitmalignomen) innerhalb der ersten drei Jahre nach der chirurgischen Entfernung der primäre Tumoren von Darmkrebspatienten im UICC Stadium I und UICC Stadium II.

Die Erfindung bezieht sich auf die Bestimmumg von Expressionsprofilen bestimmter Gene, die bei Karzinomen insbesondere gastro-intestinalen und besonders bevorzugt bei primären kolorektalen Karzinomen von Bedeutung sind. In diesem Zusammenhang lehrt die Erfindung ein Testsystem zur *(in vitro*) Detektion des Progressionsrisikos eines vorstehend genannten Karzinoms, enthaltend eine Methode zur quantitativen Messung des Expressionsprofils der Markergene ME2 und FAS in bestimmten Tumorgewebeproben sowie bioinformatische Auswertemethoden, um daraus die Wahrscheinlichkeit des Auftretens einer Progression (Lokalrezidiv, Metastasen, Zweitmalignomen) für einen Patienten zu berechnen, bei welchem ein Kolorektales Karzinom im UICC Stadium I oder UICC Stadium 11 diagnostiziert und behandelt wird. Die Markergene der Erfindung ME2 und FAS sind insbesondere in der Tabelle 1 definiert und werden durch ihre entsprechenden Sequenzen oder weitere synonyme Identifikatoren in der Tabelle charakterisiert. Dabei handelt es sich um:
mitochondrial malic enzyme 2 (NAD(+)-dependent) [Affymetrix Nummer 210154_at] SEQ_ID_1, Fas (TNF receptor superfamily, member 6) [Affymetrix Nummer 215719_x_at] SEQ_ID_2, solute carrier family 25 (mitochondrial carrier; oxoglutarate carrier), member 11 [Affymetrix Nummer 207088_s_at] SEQ_ID_3, signal transducer and activator of transcription 1, 91kDa [Affymetrix Nummer AFFX-HUMISGF3A/M97935_MB_at] SEQ_ID_4, CDC42 binding protein kinase alpha (DMPK-like) [Affymetrix Nummer 214464_at] SEQ_ID_5, glia maturation factor beta [Affymetrix Nummer 202543_s_at] SEQ_ID_6, chemokine (C-X-C motif) ligand 10 [Affymetrix Nummer 204533_at] SEQ_ID_7, mitochondrial malic enzyme 2 (NAD(+)-dependent) [Affymetrix Nummer 209397_at] SEQ_ID_8, signal transducer and activator of transcription 1, 91kDa [Affymetrix Nummer AFFX-HUMISGF3A/M97935_MA_at] SEQ_ID_9, nucleoporin 210kDa [Affymetrix Nummer 212316_at] SEQ_ID_10, dystonin [Affymetrix Nummer 212254_s_at] SEQ_ID_11, tryptophanyl-tRNA synthetase [Affymetrix Nummer 200628_s_at] SEQ_ID_12, nucleoside phosphorylase [Affymetrix Nummer 201695_s_at] SEQ_ID_13, phosphoserine aminotransferase 1 [Affymetrix Nummer 220892_s_at] SEQ_ID_14, heterogeneous nuclear ribonucleoprotein D (AU-rich element RNA binding protein 1, 37kDa) [Affymetrix Nummer 221481_x_at] SEQ_ID_15, solute carrier family 25 (mitochondrial carrier; oxoglutarate carrier), member 11 [Affymetrix Nummer 209003_at] SEQ_ID_16, methylenetetrahydrofolate dehydrogenase (NADP+ dependent) 2, methenyltetrahydrofolate cyclohydrolase [Affymetrix Nummer 201761_at] SEQ_ID_17, NADH dehydrogenase (ubiquinone) 1 alpha subcomplex, 9, 39kDa [Affymetrix Nummer 208969_at] SEQ_ID_18, transferrin receptor (p90, CD71) [Affymetrix Nummer 207332_s_at] SEQ_ID_19, 1-acylglycerol-3-phosphate O-acyltralisferase 5 (lysophosphatidic acid acyltransferase, epsilon) [Affymetrix Nummer 218096_at] SEQ_ID_20, chromatin licensing and DNA replication factor 1 [Affymetrix Nummer 209832_s_at] SEQ_ID_21, transferrin receptor (p90, CD71) [Affymetrix Nummer 208691_at] SEQ_ID_22, eukaryotic translation initiation factor 4E [Affymetrix Nummer 201435_s_at] SEQ_ID_23, peptidylglycine alpha-amidatino monooxygenase [Affymetrix Nummer 202336_s_at] SEQ_ID_24, KIT ligand [Affymetrix Nummer 207029_at] SEQ_ID_25, splicing factor, arginine/serine-rich 2 [Affymetrix Nummer 200754_x_at] SEQ_ID_26, fucosyltransferase 4 (alpha (1,3) fucosyltransferase, myeloid-specific) [Affymetrix Nummer 209892_at] SEQ_ID_27, thymidylate synthetase [Affymetrix Nummer 202589_at] SEQ_ID_28, translocated promoter region (to activated MET oncogene) [Affymetrix Nummer 201730_s_at] SEQ_ID_29, peroxiredoxin 3 [Affymetrix Nummer 201619_at] SEQ_ID_30

Die Vorhersage (Prädiktion) von Progressionen eines primären kolorektalen Karzinoms ist für den Kliniker von besonderer Relevanz, da es die weitere Behandlung des Patienten entscheidend beeinflussen kann. Finden sich keine Tumorabsiedelungen, weder in regionäre Lymphknoten noch Metastasen, handelt es sich um das UICC Stadium I oder II. Diese Tumore werden, wenn es kolorektale Karzinome sind, ausschließlich mittels Operation behandelt. Eine adjuvante Chemotherapie ist (außerhalb klinischer Studien) nicht vorgesehen. Finden sich dagegen Tumorzellen in regionären Lymphknoten (UICC Stadium III), so wird gemäß den Leitlinien der Deutschen Krebsgesellschaft und anderer internationaler Gesellschaften eine postoperative adjuvante Chemotherapie empfohlen. Durch die ajduvante Chemotherapie ergibt sich ein progressionsfreies 3 Jahres Überleben der Patienten im UICC Stadium III von etwa 69%, ohne anschließende Chemotherapie liegt das 3 Jahres progressionsfreie Überleben nur bei etwa 49%. Auch das Gesamtüberleben wird durch die adjuvanten Chemotherapie signifikant beeinflusst. Beim Rektumkarzinom ist es ebenfalls von entscheidender Bedeutung, ob bereits Tumorzellen in regionären Lymphknoten vorliegen. In diesen Fällen wird die präoperative Radiochemotherapie empfohlen, da sie das Auftreten von Lokalrezidiven im Rektum signifikant vermindert. Zudem können durch eine präoperative Radiochemotherapie signifikant mehr Patienten kontinenzerhaltend operiert werden, was bei diesen Patienten zu einer wesentlichen Verbesserung der postoperativen Lebensqualität beiträgt.

Im Zusammenhang mit dieser Erfindung bezeichnet der Begriff "kolorektales Karzinom" insbesondere polypoide, plateauförmige, ulzeröse und flächenhafte (szirrhöse) Formen, die histologisch in solide, schleimbildende oder drüsige Adenokarzinome, Siegelringzellenkarzinome, squamöse, adenosquamöse, kribriforme, plattenepithelartige oder undifferenzierte Karzinome entsprechend der WHO-Klassifikation typisiert werden. (Becker, Hohenberger, Junginger, Schlag. Chirurgische Onkologie. Thieme, Stuttgart 2002)

Im Zusammenhang mit der Erfindung umfasst der Begriff "Genexpressionsprofil" sowohl die Bestimmung von "Expressionsprofilen", als auch der einzelnen "Expressionsniveaus" der entsprechenden Gene. Der Begriff "Expressionsniveau", als auch der Begriff "Expressionsprofil" umfasst erfindungsgemäß sowohl die Quantität des Genprodukts, als auch seine qualitative Veränderungen wie z.B. Methylierungen, Glykosylierungen, Phosphorylierungen u.s.w. Somit wird bei der Bestimmung des "Expressionsprofils" im Zusammenhang mit der Erfindung im Wesentlichen auf die Quantität der entsprechenden Genprodukte (RNA/ Protein) geachtet. Das Expressionsniveau wird gegebenenfalls mit dem anderer Individuen verglichen. Entsprechende Ausführungsbeispiele sind im experimentellen Teil belegt und auch insbesondere in den Tabellen dargestellt.

Die Bestimmung der Expressionsprofile der hierin dargestellten Gene (Genabschnitte) wird insbesondere in Geweben und/oder einzelnen Zellen des Gewebes durchgeführt. Verfahren zur Bestimmung des Expressionsprofils umfassen daher (im Sinne dieser Erfindung) z.B. *in situ*-Hybridisierungen, PCR basierte Methoden (z.B. Taqman) oder Mikroarray basierte Verfahren (siehe auch experimenteller Teil der Erfindung).

In einer besonderen Ausführungsform umfasst die Erfindung das oben genannte Verfahren, wobei das Expressionsprofil der 2 Markergene ME2 und FAS, die durch SEQ ID NO 1 bis SEQ ID NO 2 eindeutig beschrieben sind, bestimmt wird.

Es umfasst die Offenbarung das oben genannte Verfahren, wobei das Expressionsprofil von genau neun Markergenen, wie in SEQ ID NO 1 bis SEQ ID NO 9 dargestellt, bestimmt wird.

Es umfasst die Offenbarung das oben genannte Verfahren, wobei das Expressionsprofil von genau fünf Markergenen, wie in SEQ ID NO 1 bis SEQ ID NO 5 dargestellt, bestimmt wird.

Wie im Weiteren definiert wird, umfasst der Begriff Markergene im Sinne dieser Erfindung nicht nur die spezifischen Gensequenzen (oder die entsprechenden Genprodukte) wie in den spezifischen Nucleotidsequenzen dargestellt, sondern auch Gensequenzen, die eine hohe Homologie zu diesen Sequenzen aufweisen. Weiterhin sind die revers komplementären Sequenzen der definierten Markergene eingeschlossen. Hochhomologe Sequenzen umfassen Sequenzen, die mindestens 80%, vorzugsweise mindestens 90%, am meisten bevorzugt mindestens 95% homolog zu den in den SEQ ID NOS: 1 bis 30 dargestellten Sequenzen sind.

Im Kontext dieser Erfindung umfassen solche hochhomologen Sequenzen auch Sequenzen, die Genprodukte (z.B. RNA oder Proteine) codieren, welche zumindest 80% identisch zu den definierten Genprodukten der SEQ ID NOS: 1 bis 30 sind. Der Begriff Markergen im Zusammenhang mit dieser Erfindung umfasst erfindungsgemäß ein Gen oder einen Genabschnitt, der mindestens 90 % homolog, mehr bevorzugt mindestens 95 % homolog, mehr bevorzugt mindestens 98 % homolog, am meisten bevorzugt mindestens 100 % homolog zu den in den SEQ ID NO 1 bis SEQ ID NO 30 dargestellten Sequenzen in Form von Desoxyribonnukleotiden oder entsprechenden Ribonunkleotiden bzw. den daraus abgeleiteten Proteinen ist.

Unter einem aus den 30 Markergenen (definiert in den SEQ ID NOs 1 bis 30, in Tabelle 1) abgeleiteten Proteinen wird in dieser Offenbarung ein Protein, ein Proteinfragment oder ein Polypeptid verstanden, das im nativen Leserahmen (in frame) translatiert wurde.

Die Sequenzidentität kann konventionell durch Verwendung von Computerprogrammen wie z. B. dem FASTA-Programm (W. R. Pearson (1990) Rapid and Sensitive Sequence Comparison with FASTP and FASTA Methods in Enzymology 183:63 - 98.), download z.B. als Service des EBI in Hinxton, bestimmt werden. Bei der Anwendung von FASTA oder einem anderen Sequenz-Alignment-Programm zur Bestimmung, ob eine bestimmte Sequenz beispielsweise zu 95% identisch ist mit einer Referenzsequenz der vorliegenden Erfindung, werden die Parameter vorzugsweise so eingestellt, dass der Prozentanteil der Identität über die gesamte Länge der Referenzsequenz berechnet wird und dass Homologielücken (so genannte gaps) von bis zu 5% der Gesamtzahl der Nukleotide in der Referenzsequenz erlaubt sind. Wichtige Programmparameter wie z.B. GAP PENALTIES und KTUP werden auf den Standardwerten belassen.

Es können die entsprechenden Markergene in Tumor- Proben, bestimmt werden. Die erfindungsgemäßen Ergebnisse können ebenfalls durch die Untersuchung von fixieitern Tumorgewebe zum Beispiel Paraffinmaterial, erhalten werden. In fixiertem Material werden insbesondere auch vorzugsweise andere Detektionsverfahren zum Nachweis der Gene/Genexpressionsprodukte genutzt, z.B. RNA spezifische Primer im Rahmen einer real-time PCR.

Wie auch in den Ausführungsbeispielen der Erfindung dargestellt, wird das Expressionsprofil der hier offenbarten 2 Markergene vorzugsweise, durch die Messung der Quantität der mRNA der Markergene bestimmt. Diese Quantität der mRNA der Markergene kann z.B. mittels Genchiptechnologie, (RT-) PCR (zum Beispiel auch an fixiertem Material), Northern Hybridisierung, Dot-Blotting oder in situ Hybridisierung bestimmt werden.

Die in der vorliegenden Erfindung besonders vorzugsweise verwendete Mikroarray Technologie erlaubt die gleichzeitige Messung des mRNA Expressionsniveaus von vielen Tausend Genen und stellt somit ein wichtiges Hilfsmittel dar, um differentielle Expression zwischen zwei biologischen Proben oder Gruppen von biologischen Proben zu bestimmen. Analysen können aber auch, wie dem Fachmann bekannt, durch einzelne reverse Transkriptase-PCR, kompetetive PCR, "real time PCR", "differential display RT-PCR, Northern.Blot-Analyse und andere verwandte Verfahren durchgeführt werden.

Am Besten ist es jedoch, die komplementäre DNA (cDNA) bzw. komplementäre RNA (cRNA), welche ausgehend von der zu untersuchenden RNA produziert wird, mit Hilfe von Mikroarrays zu untersuchen. Eine große Zahl von verschiedenen Arrays sowie deren Herstellung sind dem Fachmann bekannt und beispielsweise in den US Patenten 5,445,934; 5,532,128; 5,556,752; 5,242,974; 5,384,261; 5,405,783; 5,412,087; 5,424,186;5,429,807; 5,436,327; 5,472,672; 5,527,681; 5,529,756; 5,54533 1; 5,554,501; 5,561,071;5,571,639; 5,593,839; 5,599,695; 5,624,711; 5,658,734; und 5,700,637 dargestellt.

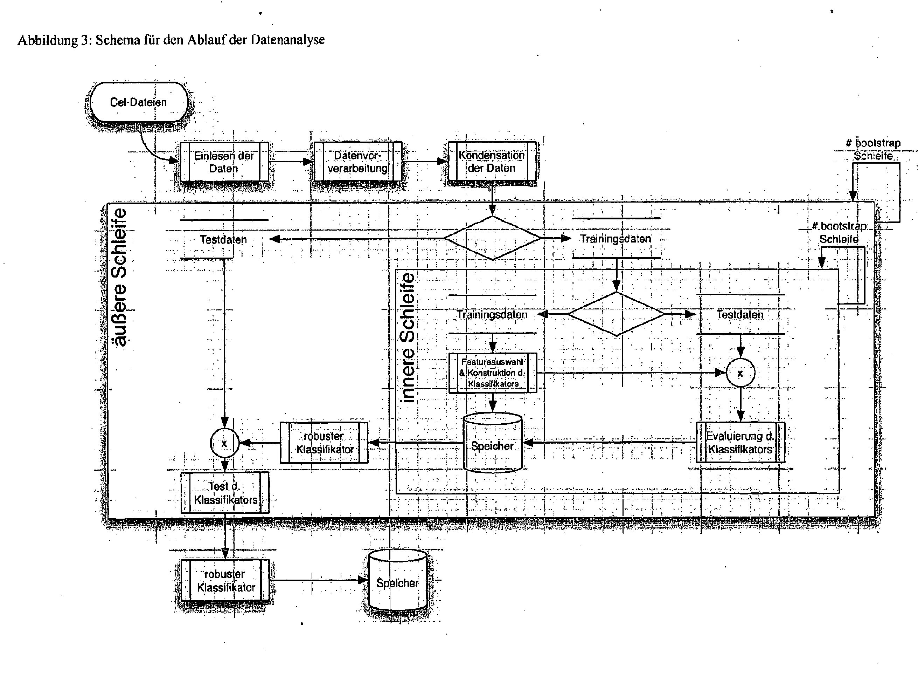

In einer weiteren Ausführungsform umfasst die Offenbarung die wohl definierte Abfolge von Analysenschritten welche letztendlich zur Entdeckung der Markergensignaturen führt, mit denen die Test- von der Kontrollgruppe unterscheidenden werden kann. Diese bisher in dieser Form noch nicht beschriebene Methode setzt sich wie in den Ausführungsbeispielen detaillierter beschrieben und in Abbildung 3 skizziert wie folgt zusammen:

Die Rohdaten der Biochips werden zunächst mit FARMS wie von Hochreiter (2006), Bioinformatics 22(8):943-9, gezeigt kondensiert und anschließend in einem zweifach geschachtelten Bootstrap-Ansatz, [Efron (1979) Bootstrap Methods - Another Look at the Jackknifing, Ann. Statist. 7, 1-6. in der äußeren Schleife in Test- und Trainigsdaten aufgeteilt. In der inneren Bootstrap-Schleife wird für die Trainingsdaten durch eine Decision-Tree-Analyse die Featurerelevanz aus den Daten extrahiert. Dazu werden in mehreren Bootstrap-Iterationen jeweils eine bestimmte Zahl von zu klassifizierenden Proben zufällig gezogen und der Einfluss eines Features aus dessen Beitrag zum Klassifikationsfehler ermittelt: Steigt der Fehler durch Permutieren der Werte eines Features, während die Werte aller anderen Features unverändert blieben, so wird dieses stärker gewichtet. Anhand einer Häufigkeitstabelle werden die am häufigsten gewählten Features ermittelt und für die Klassifikation der Testdaten durch eine Support-Vector-Machine oder anderweitige dem Fachmann bekannte Klassifikationsalgorithmen wie z.B. Kassifikations- und Regressionsbäume, Penalized Logistic Regression, Sparse Linear Discriminant Analysis, Fisher Linear Discriminant Analysis, K-Nearest Neighbors, Shrunken Centroids und Artificial Neural Networks in der äußeren Bootstrap-Schleife verwendet.

Ein Feature stellt in diesem Zusammenhang einen bestimmten Messpunkt für ein zu untersuchendes Gen dar, welches sich auf der Biochipoberfläche befindet und mit der zu untersuchenden markierten Probe hybridisiert und somit ein Intenstätssignal liefert. Die vorliegende Erfindung betrifft ebenfalls ein Kit zur Durchführung der hier beschrieben Verfahren, wobei das Kit spezifische DNA- oder RNA-Sonden, Primer (auch Primerpaare), Antikörper, Aptamere zur Bestimmung der Markergene, die in SEQ ID NO: 1 bis 2 dargestellt sind, oder zur Bestimmung von mindestens einem Genprodukt der 2 Markergene die von den Sequnzen in SEQ ID NO: 1 bis 2 kodiert werden, umfasst. Der Kit ist vorzugsweise ein diagnostischer Kit. Als Kit im Sinne der Erfindung wird auch ein beliebiges Mikroarray oder im Speziellen ein "Affymetrix-Genchip" bezeichnet. Der Kit kann alle oder einige der für die Durchführung des Assays benötigten Materialien sowie die erforderlichen Instruktionen enthalten.

Gegenstand der Offenbarung sind auch Darstellungen der Markergensignaturen, welche für die Behandlung, die Diagnose und die Prognose der oben genannten Erkrankungen nützlich sind. Diese Genprofildarstellungen sind auf Medien reduziert, welche maschinenlesbar sind wie z.B. computerlesbare Medien (magnetische Medien, optische Medien u.s.w.). Die Gegenstände der Offenbarung können CD ROMs sein, welche Computerprogramme für den Vergleich mit dem gespeicherten 30-Gen-Expressionsprofil, welches oben beschrieben wurde, enthalten. Ebenso können die erfindungsgemäßen Gegenstände digital gespeicherte Expressionsprofile enthalten, sodass diese mit Expressions daten von Patienten verglichen werden können. Alternativ können solche Profile in einem anderen gegenständlichem Format gespeichert werden. Eine grafische Darstellung ist beispielsweise ein solches Format.

Im Folgenden soll die Erfindung genauer auf der Basis der Sequenzen, Tabellen und Beispiele beschrieben werden, ohne darauf begrenzt zu werden.

Die Tabellen zeigen:
**Tabelle 1a** enthält die 30 Markergene, die differentiell zwischen Patienten mit und ohne Progression des primär aufgetretenen kolorektalen Karzinoms exprimiert sind, wenn im Validations-Bootstrap in jeder Iteration ein Datensatz als Testset verwandt wird.
**Tabelle 1b** enthält die 30 Markergene, die differentiell zwischen Patienten mit und ohne Progression des primär aufgetretenen kolorektalen Karzinoms exprimiert sind, wenn im Validations-Bootstrap in jeder Iteration zwei Datensätze als Testset verwandt werden.
**Tabelle 1c** enthält die 30 Markergene, die differentiell zwischen Patienten mit und ohne Progression des primär aufgetretenen kolorektalen Karzinoms exprimiert sind, wenn im Validations-Bootstrap in jeder Iteration drei Datensätze als Testset verwandt werden.
**Tabelle 2a** zeigt die Maßzahlen der Klassifikation des fünfjährigen progressionsfreien Überlebens für das ausgewählte Patientenkollektiv (insgesamt 55 davon 26 mit Progression) in Abhängigkeit von der verwendeten Markergenanzahl, wenn im Validations-Bootstrap in jeder Iteration ein Datensatz als Testset verwandt wird.
**Tabelle 2b** zeigt die Maßzahlen der Klassifikation des fünfjährigen progressionsfreien Überlebens für das ausgewählte Patientenkollektiv (insgesamt 55 davon 26 mit Progression) in Abhängigkeit von der verwendeten Markergenanzahl, wenn im Validations-Bootstrap in jeder Iteration zwei Datensätze als Testset verwandt werden.
**Tabelle 2c** zeigt die Maßzahlen der Klassifikation des fünfjährigen progressionsfreien Überlebens für das ausgewählte Patientenkollektiv (insgesamt 55 davon 26 mit Progression) in Abhängigkeit von der verwendeten Markergenanzahl, wenn im Validations-Bootstrap in jeder Iteration drei Datensätze als Testset verwandt werden.

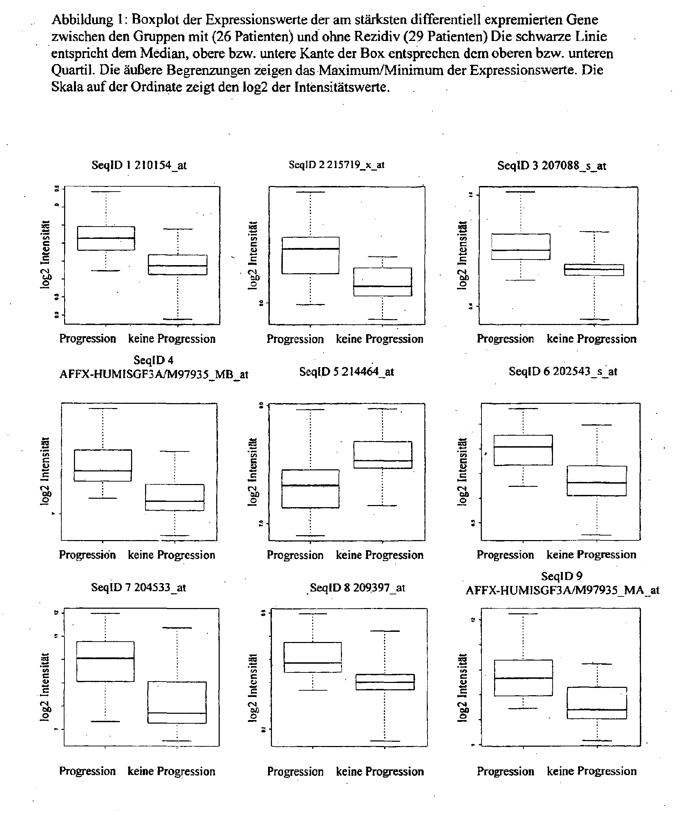

**Abbildung 1** zeigt den Boxplot der Expressionwerte der besten 10 Gene aus der Markergenliste für die Patientengruppen mit oder ohne Progression innerhalb der ersten fünf Jahre nach der Operation, wenn im Validations-Bootstrap in jeder Iteration zwei Datensätze als Testset verwandt werden.

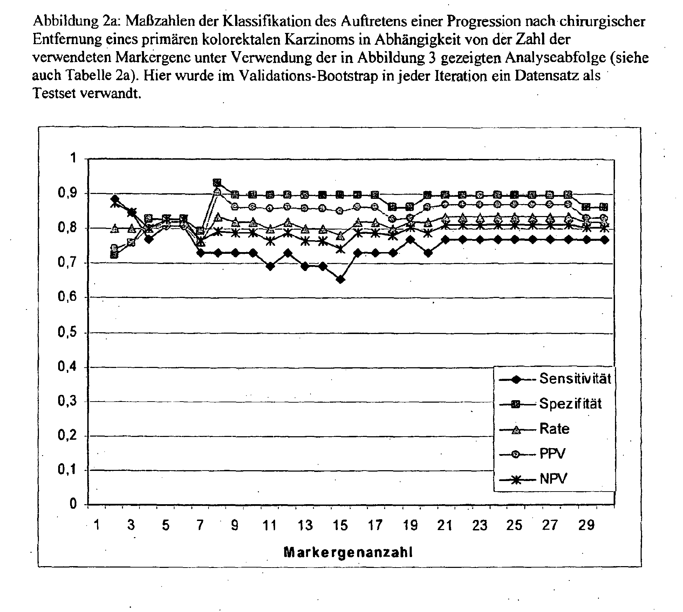

**Abbildung 2a** zeigt die Maßzahlen der Klassifikation des Auftretens einer Progression innerhalb von fünf Jahren nach der primären Diagnose eines kolorektalen Karzinoms in Abhängigkeit von der verwendeten Markergenanzahl, wenn im Validations-Bootstrap in jeder Iteration ein Datensatz als Testset verwandt wird.

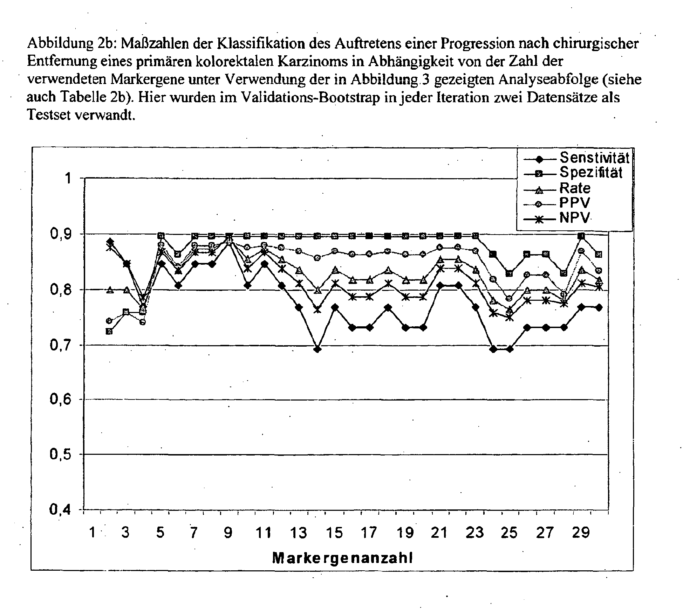

**Abbildung 2b** zeigt die Maßzahlen der Klassifikation des Auftretens einer Progression innerhalb von fünf Jahren nach der primären Diagnose eines kolorektalen Karzinoms in Abhängigkeit von der verwendeten Markergenanzahl, wenn im Validations-Bootstrap in jeder Iteration zwei Datensätze als Testset verwandt werden.

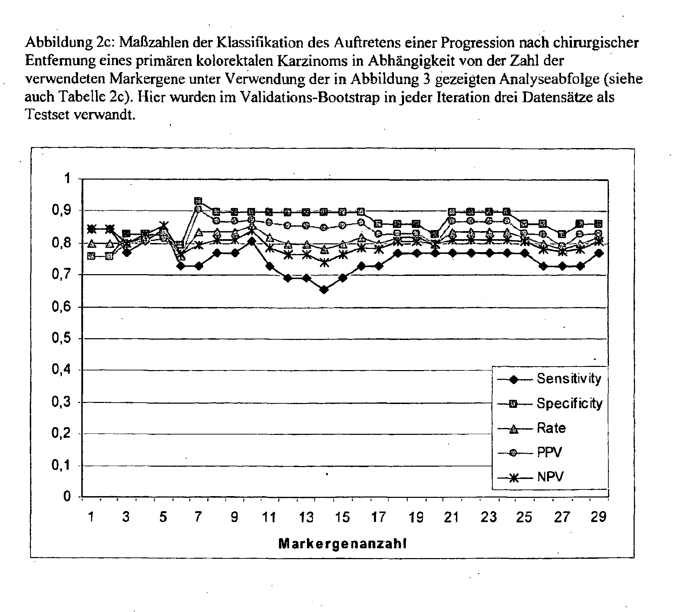

**Abbildung 2c** zeigt die Maßzahlen der Klassifikation des Auftretens einer Progression innerhalb von fünf Jahren nach der primären Diagnose eines kolorektalen Karzinoms in Abhängigkeit von der verwendeten Markergenanzahl, wenn im Validations-Bootstrap in jeder Iteration drei Datensätze als Testset verwandt werden.
**Abbildung 3** zeigt schematisch das methodische Vorgehen, welches zur Erstellung des Markergenprofils führt.

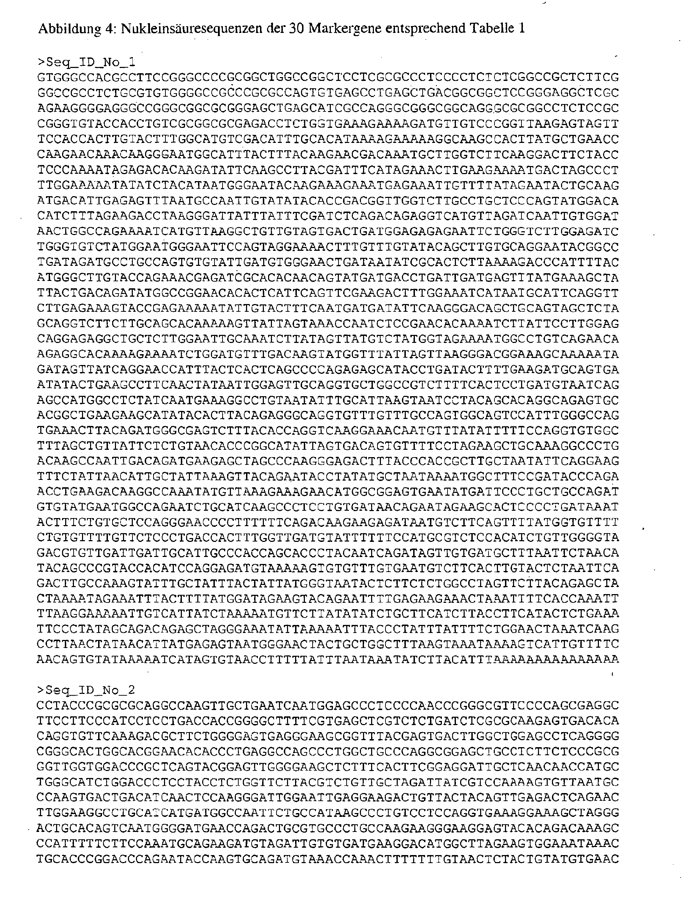

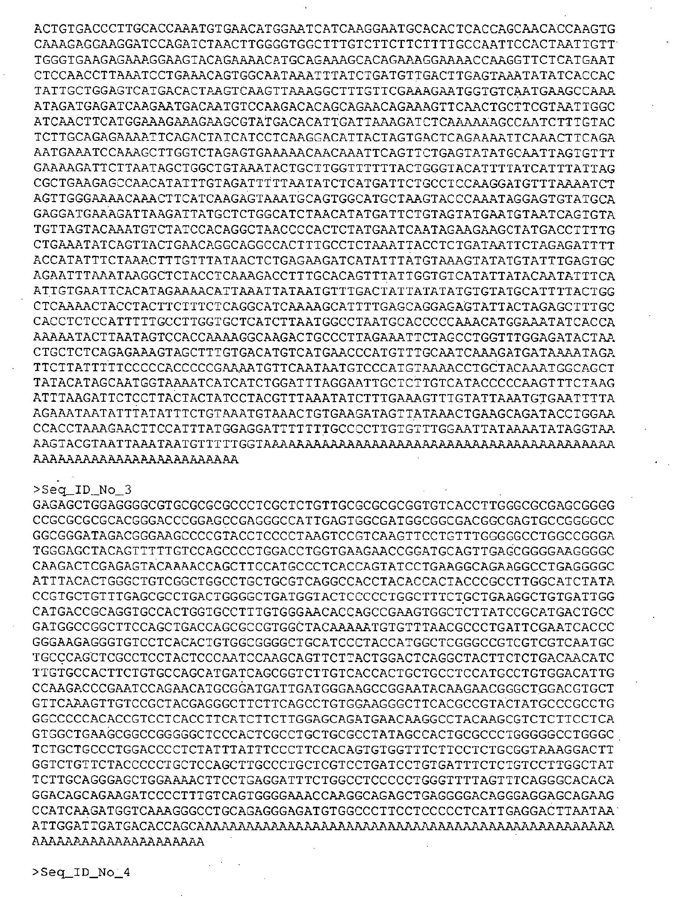

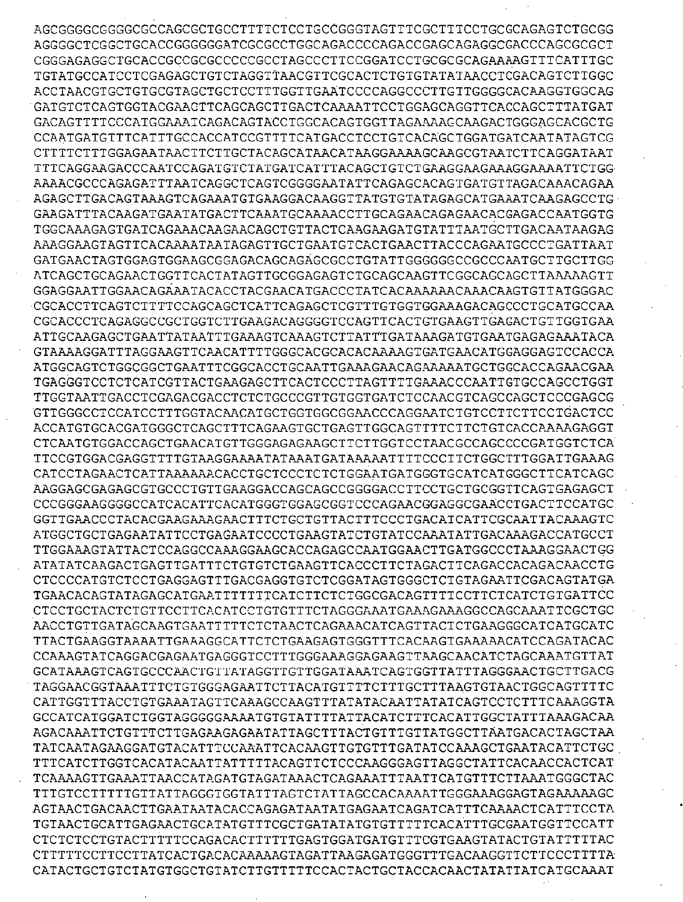

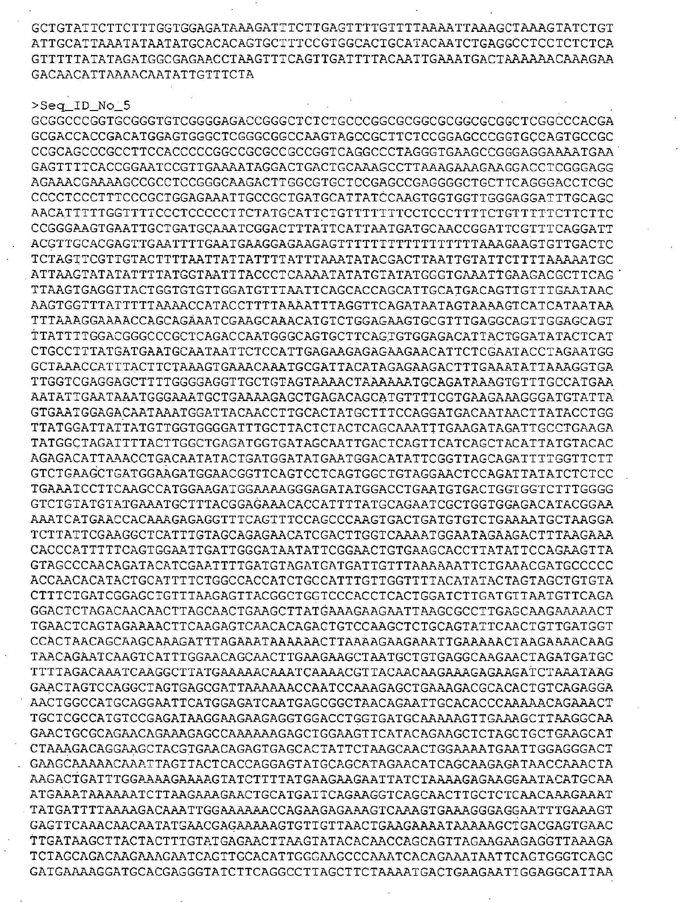

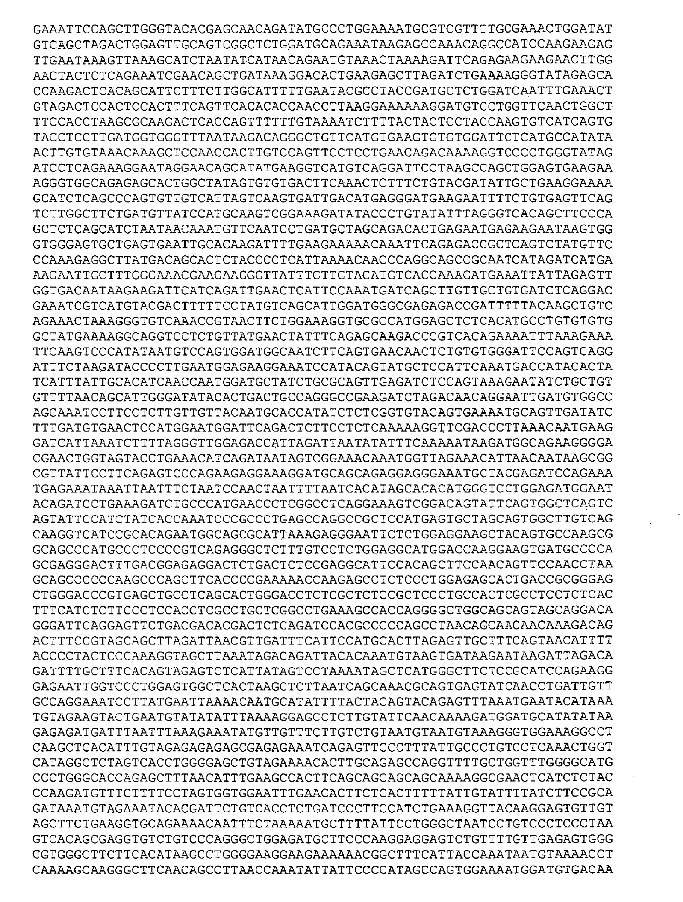

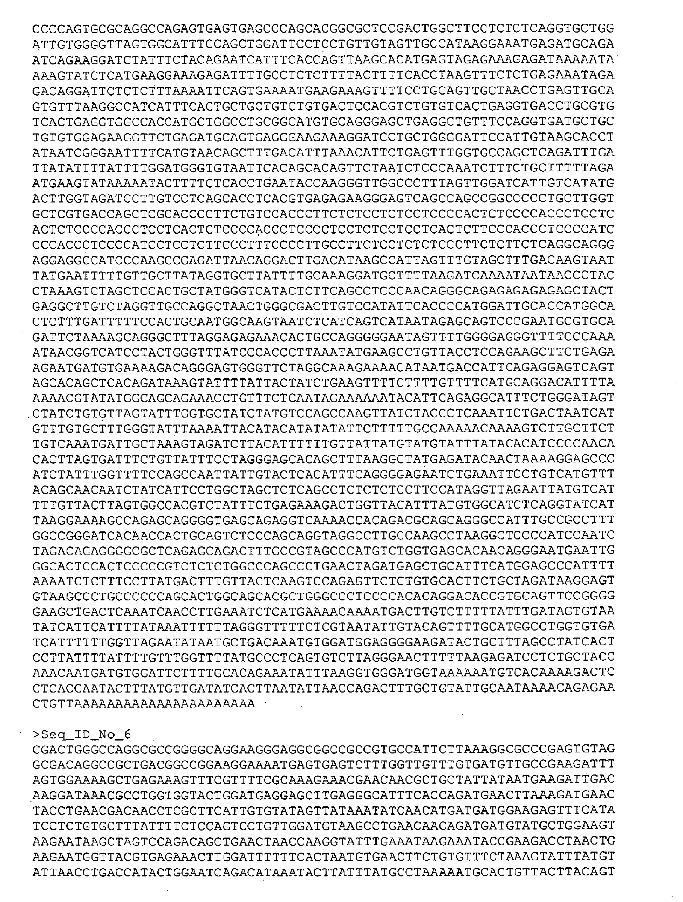

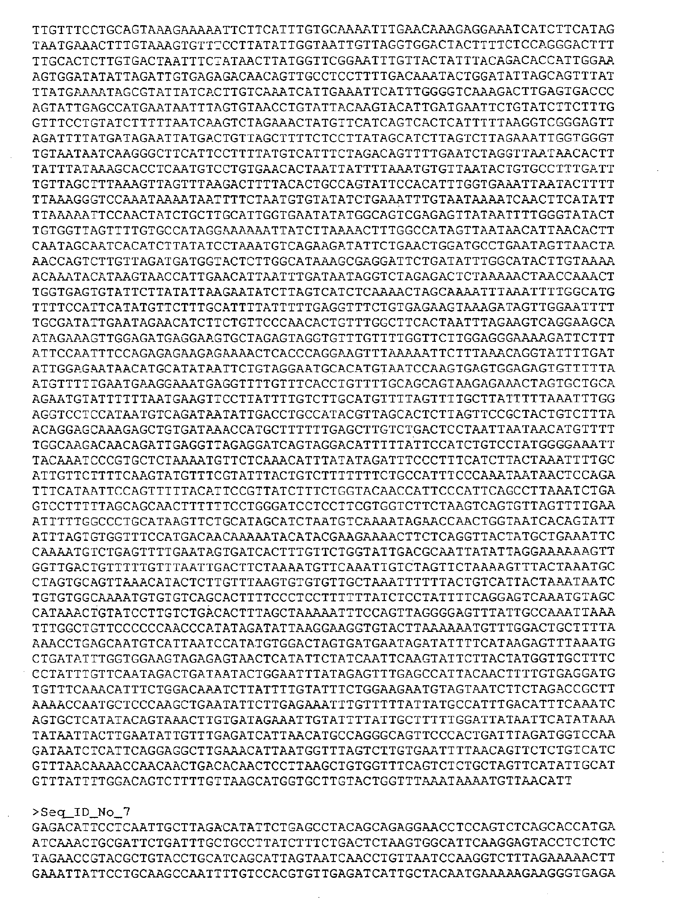

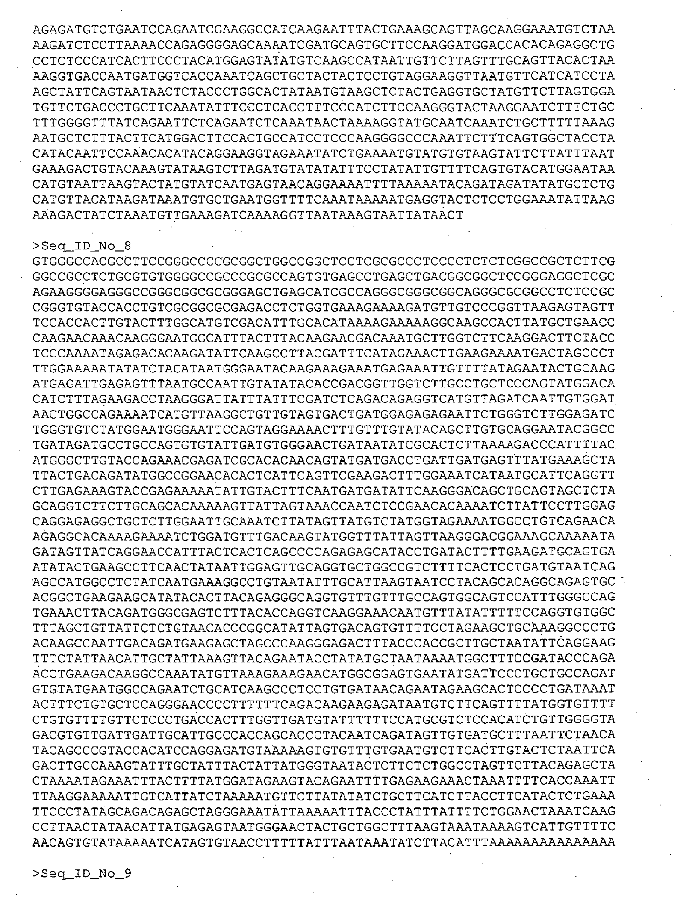

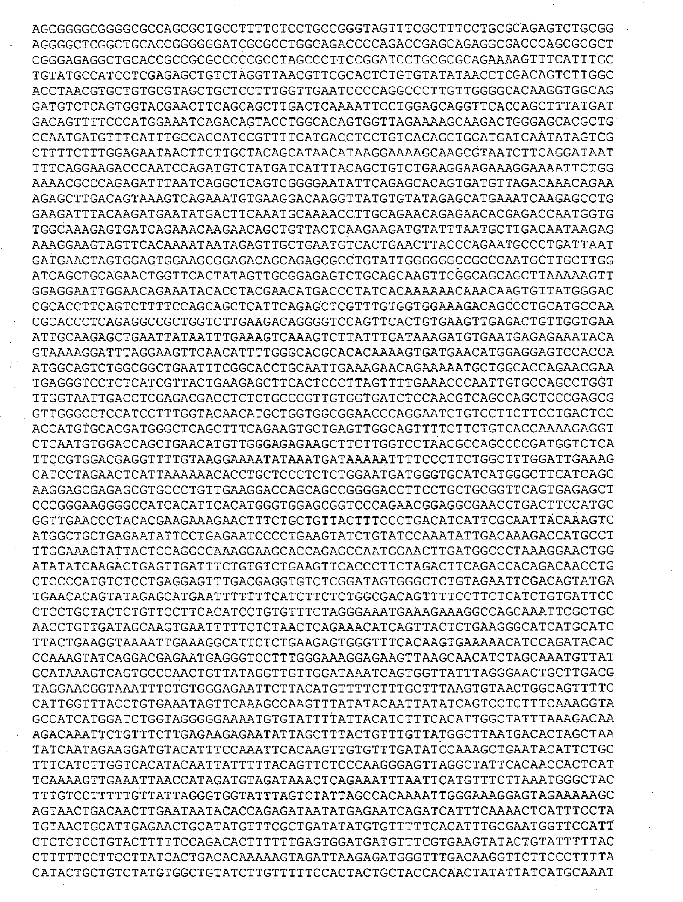

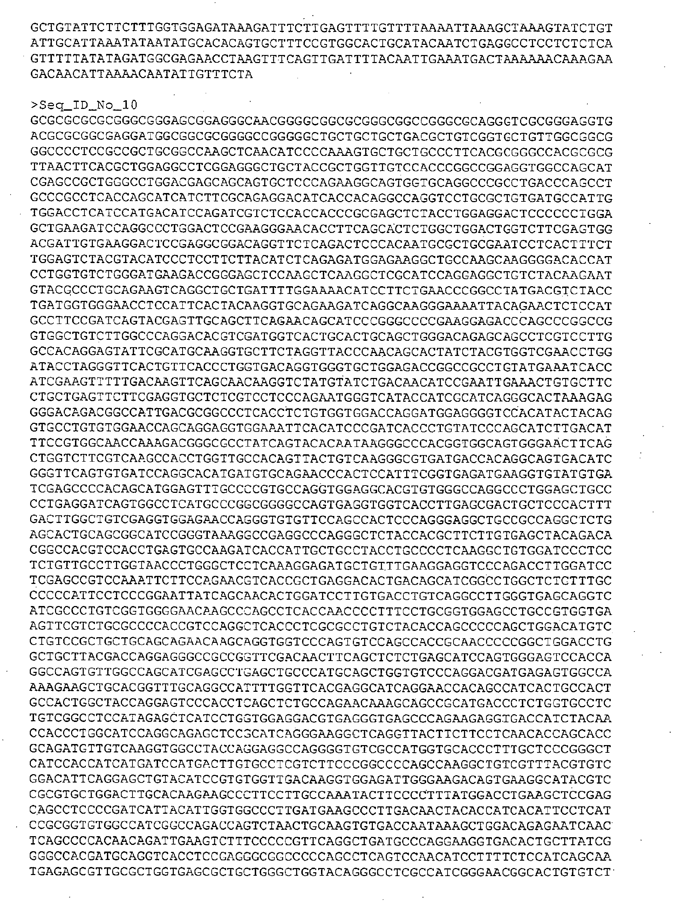

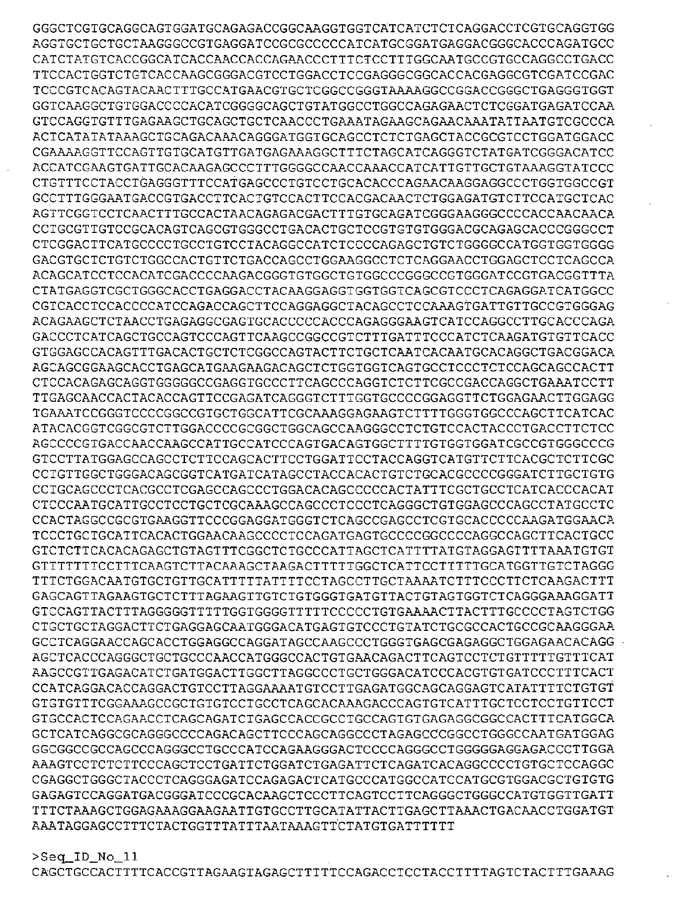

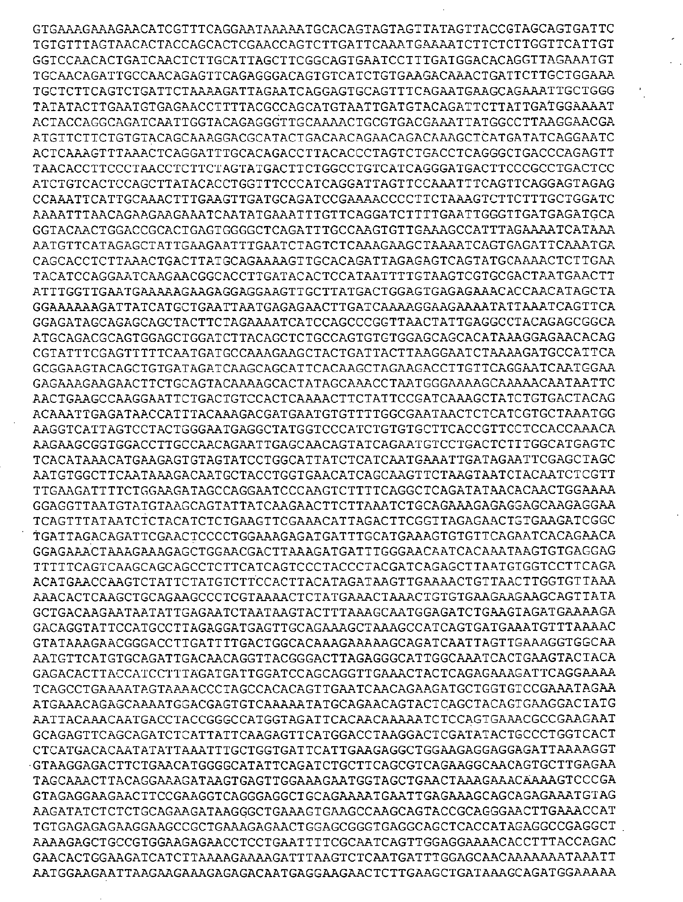

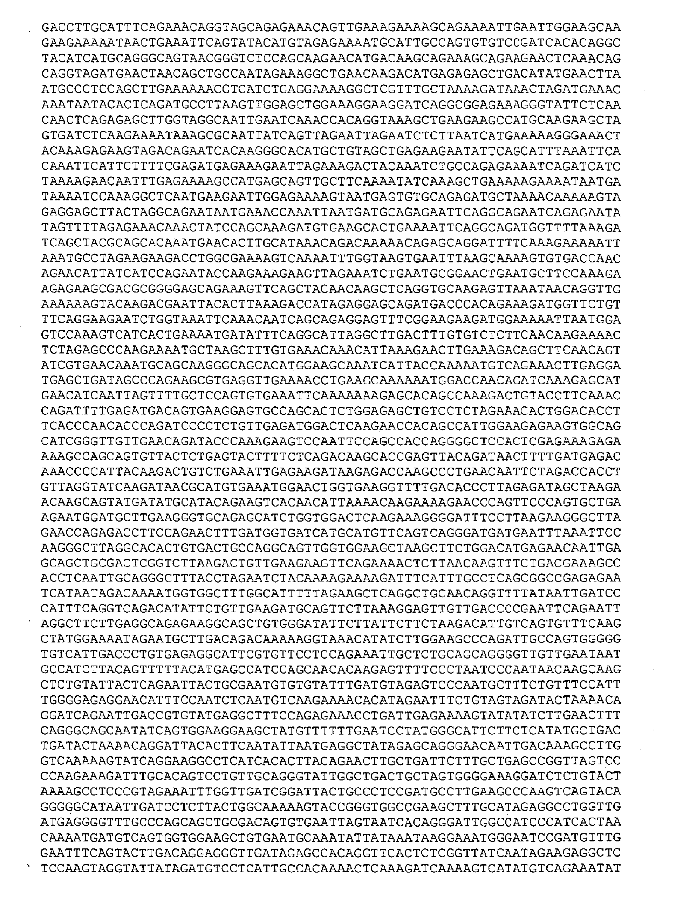

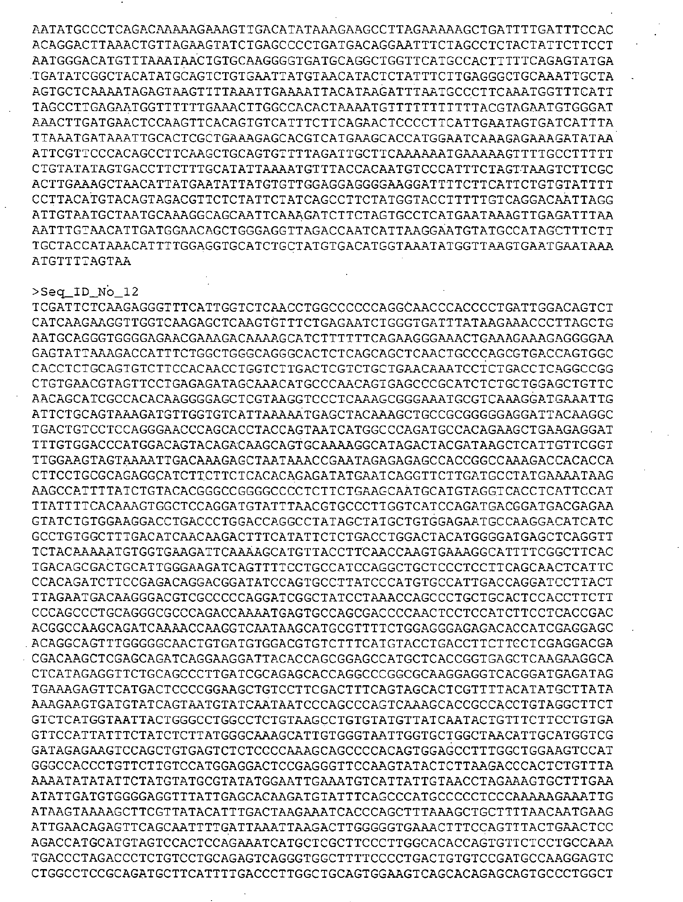

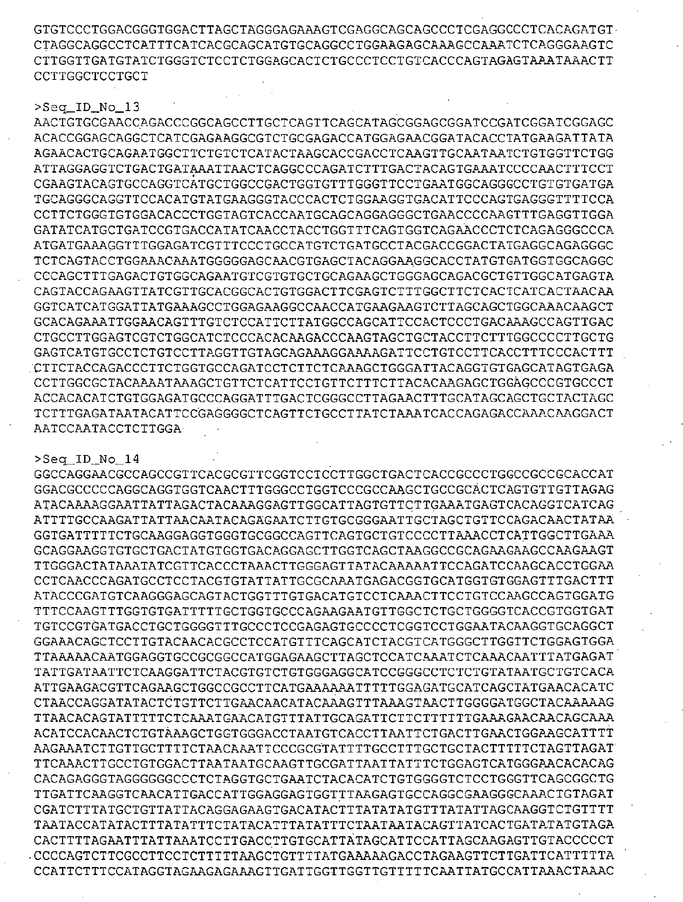

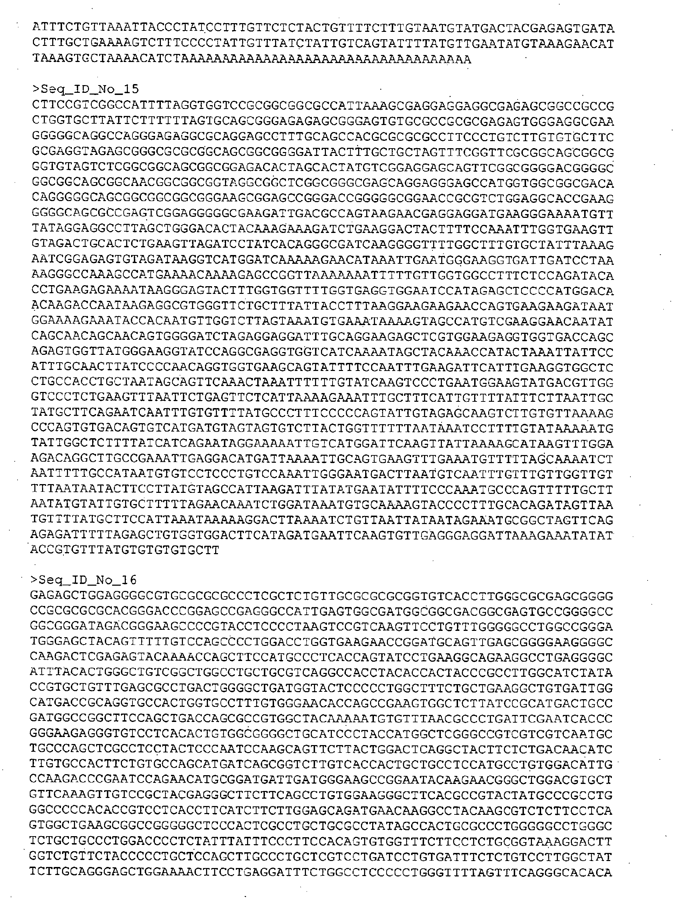

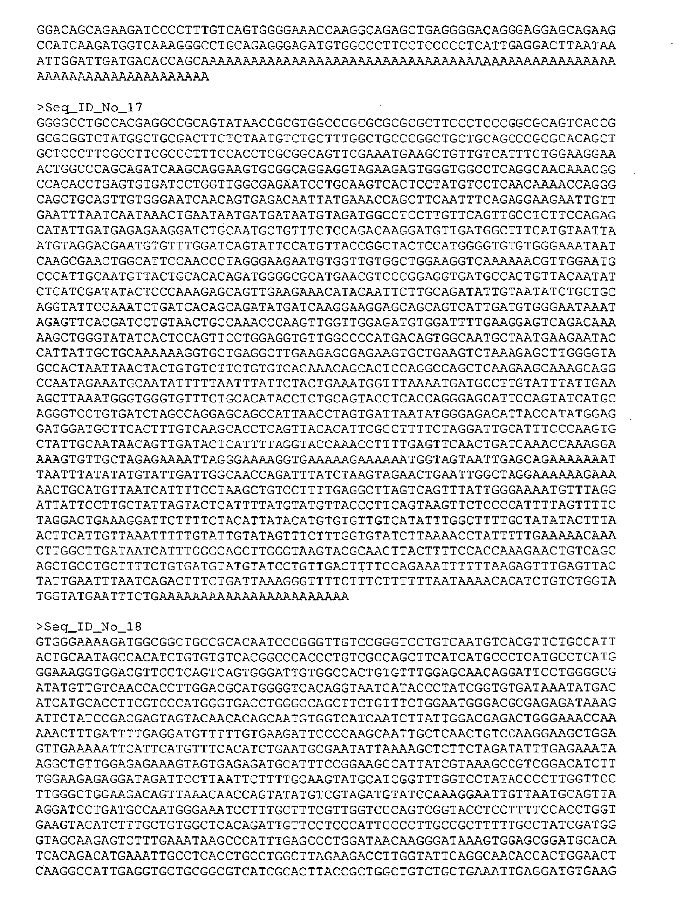

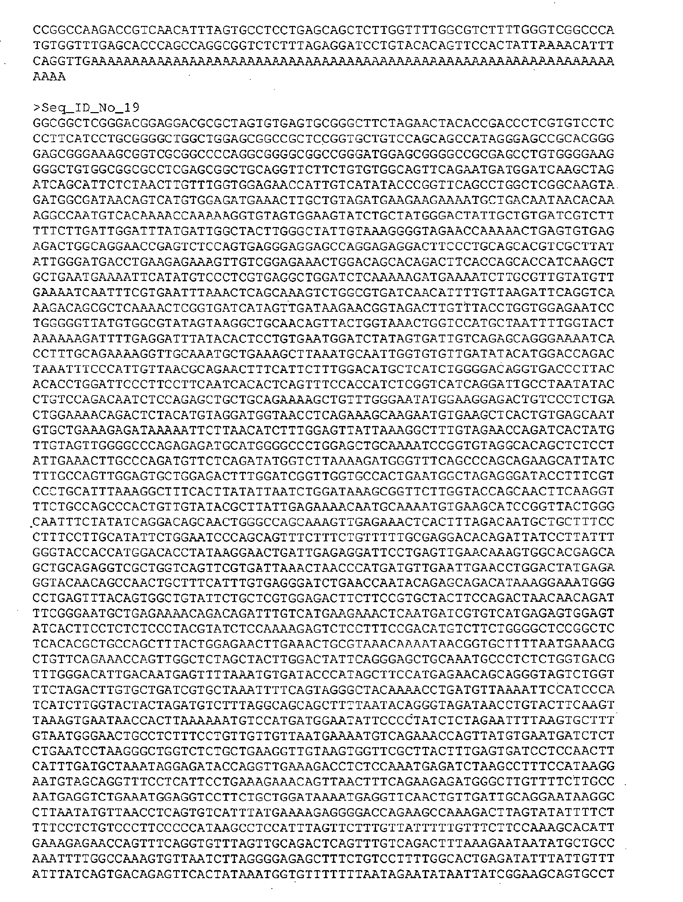

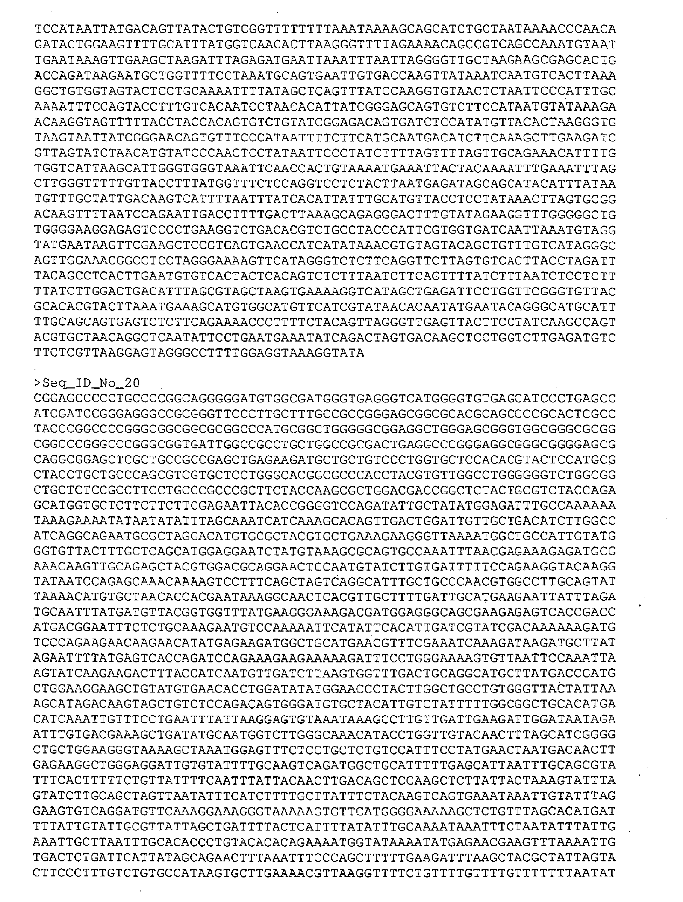

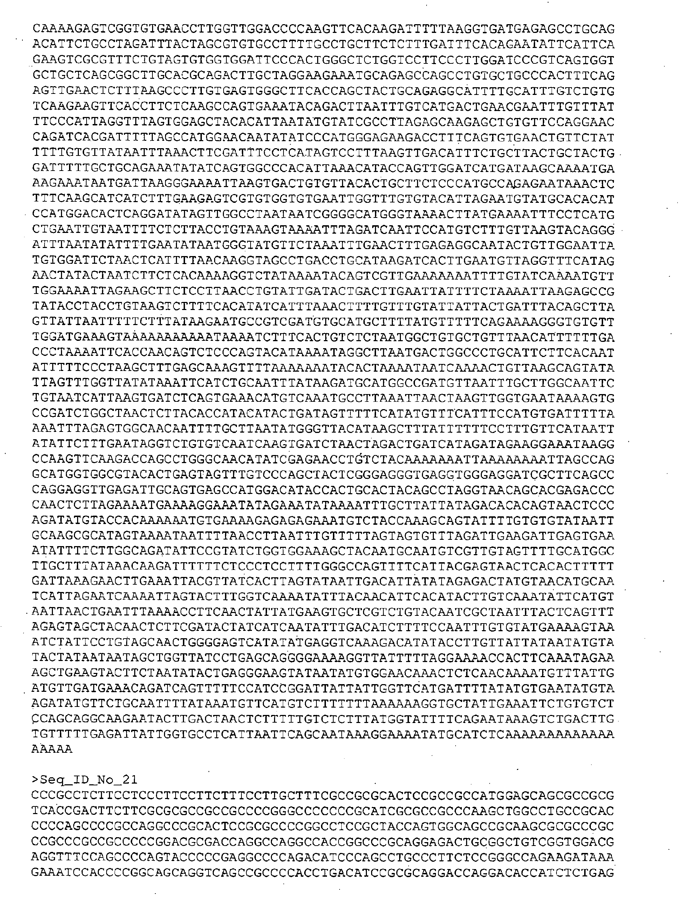

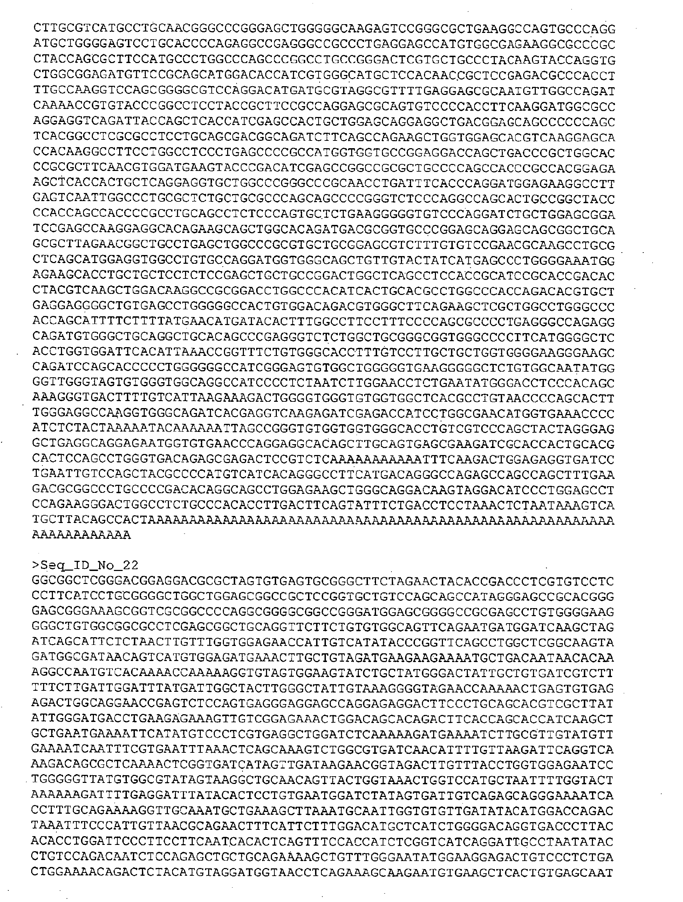

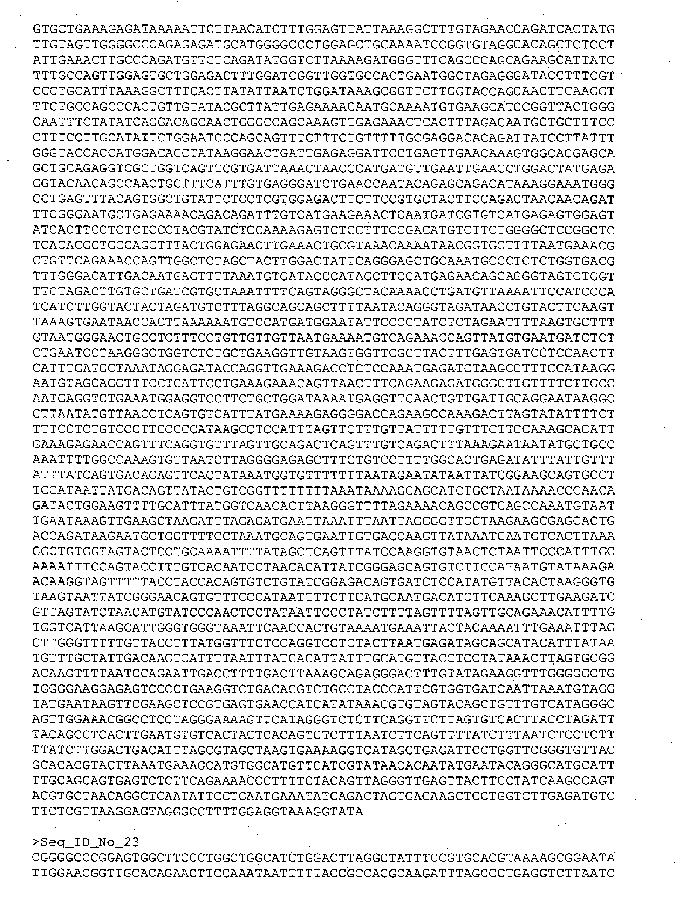

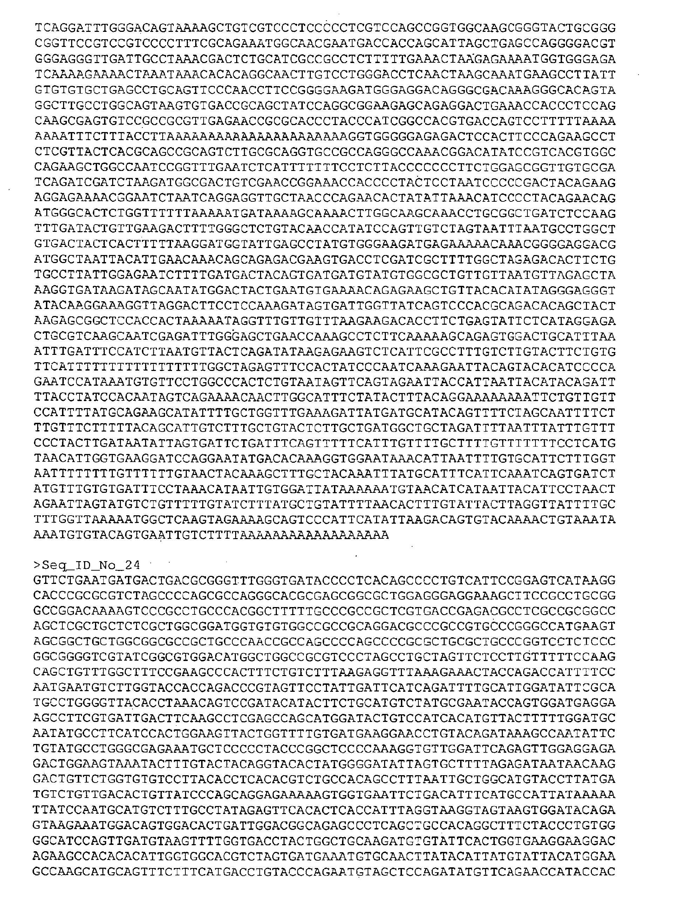

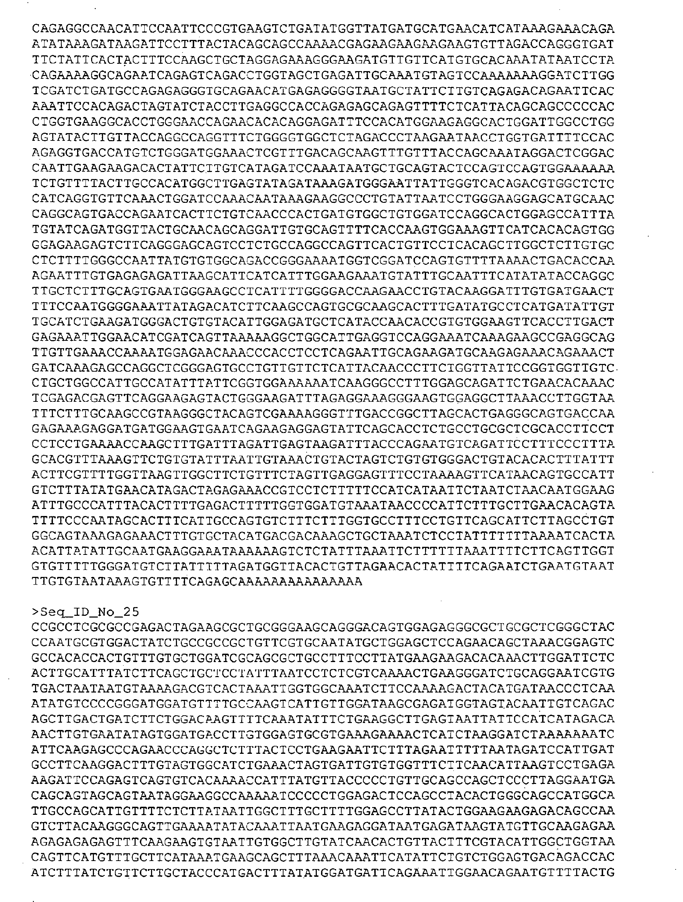

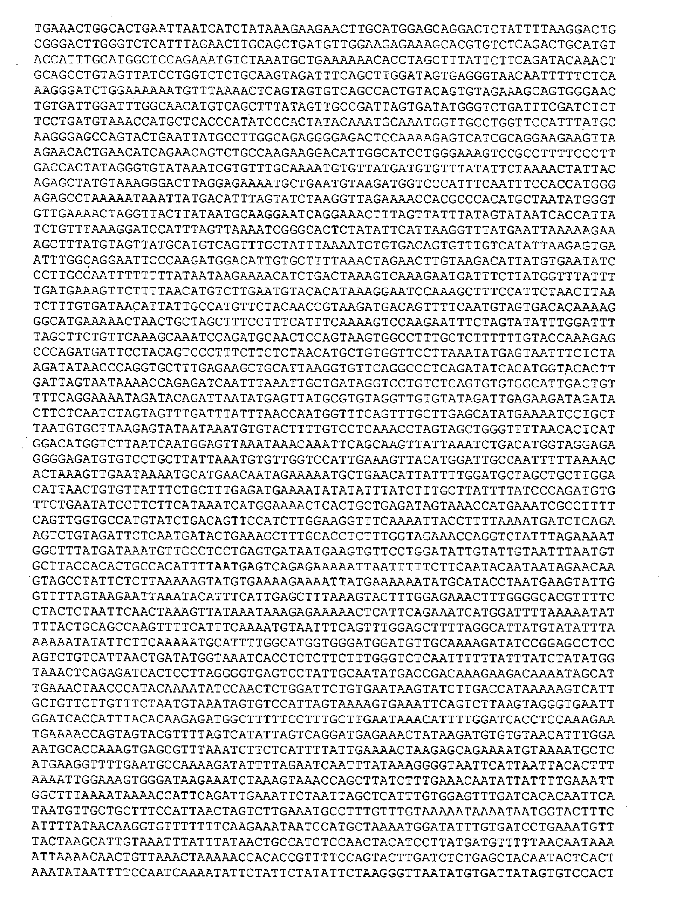

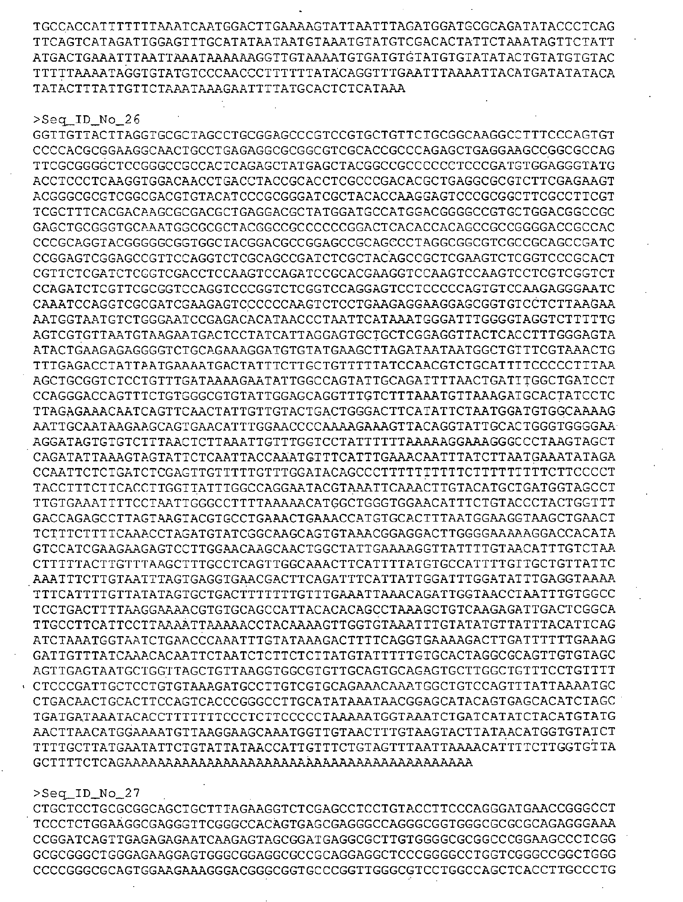

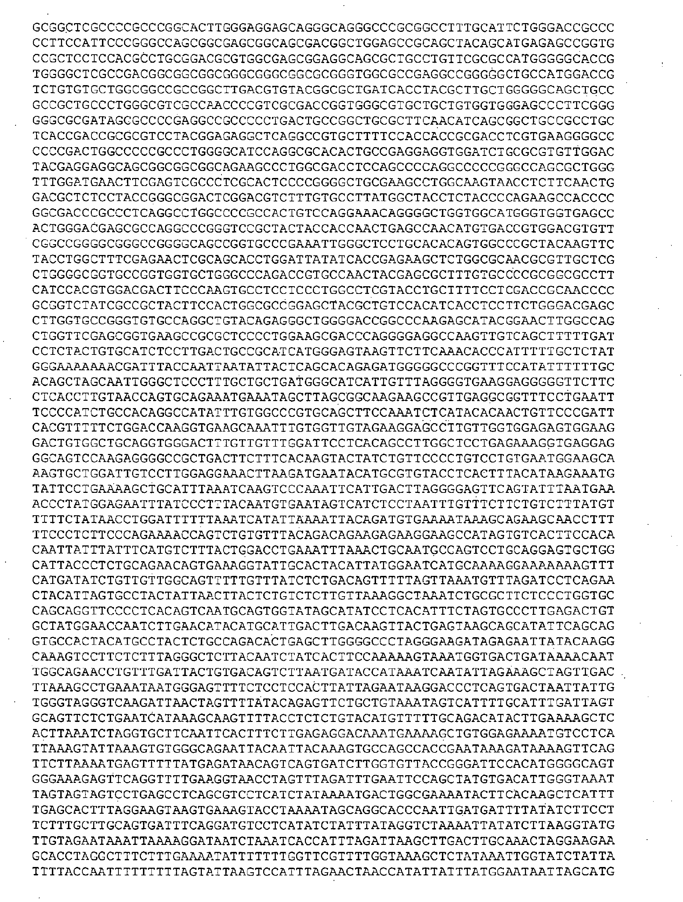

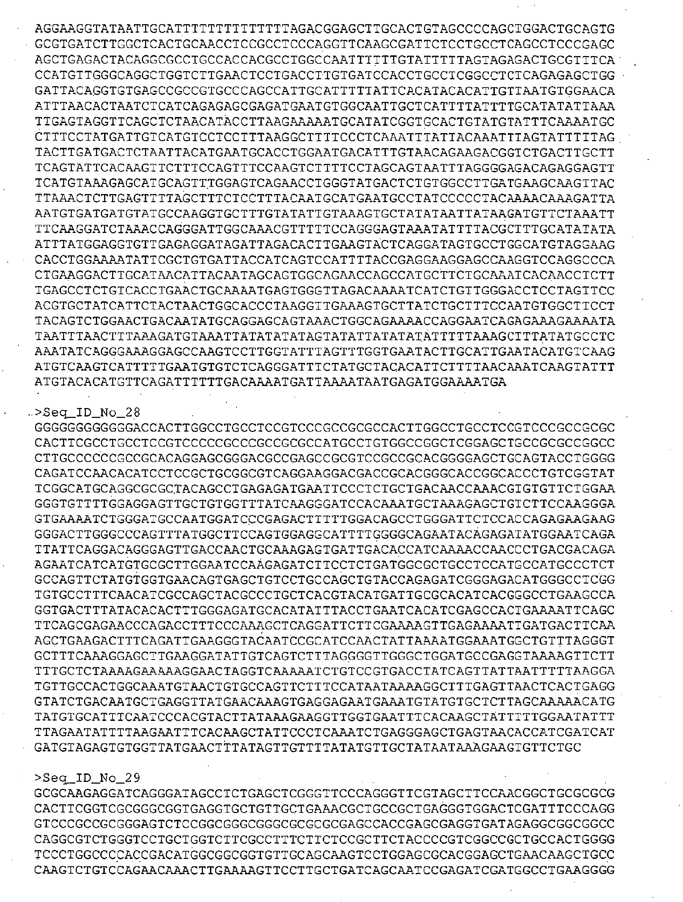

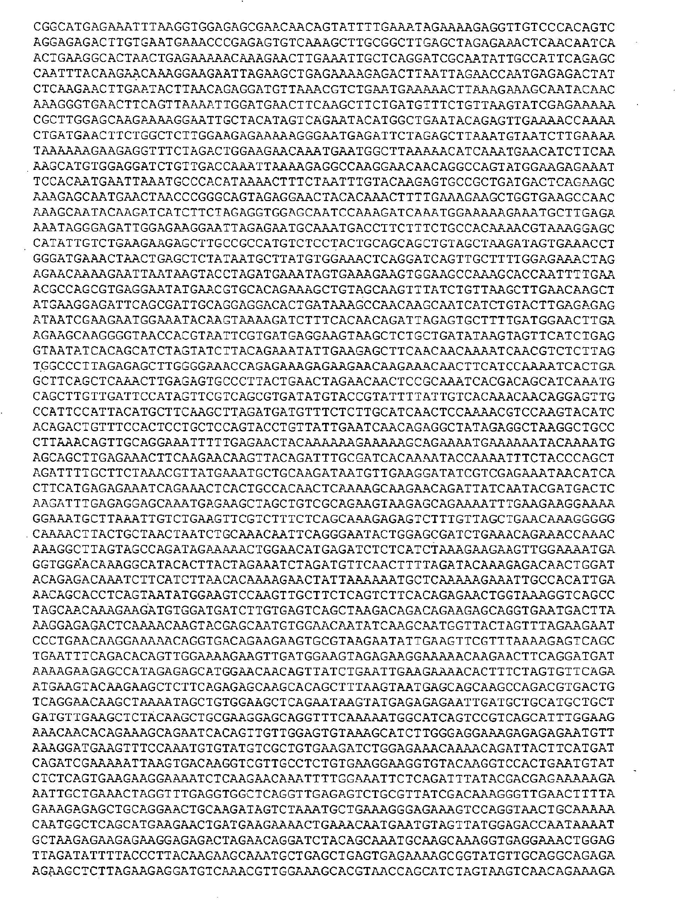

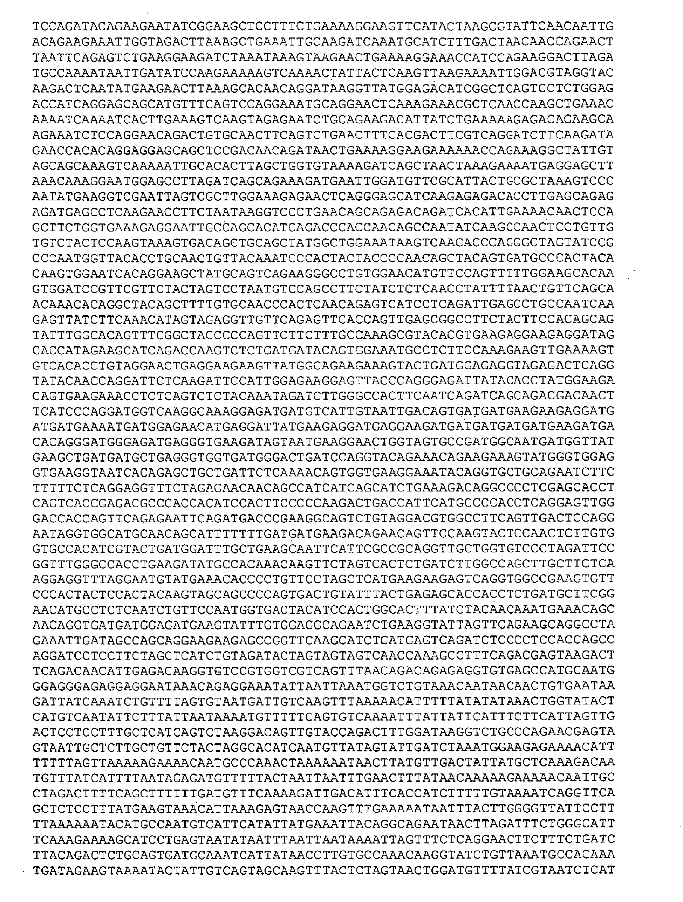

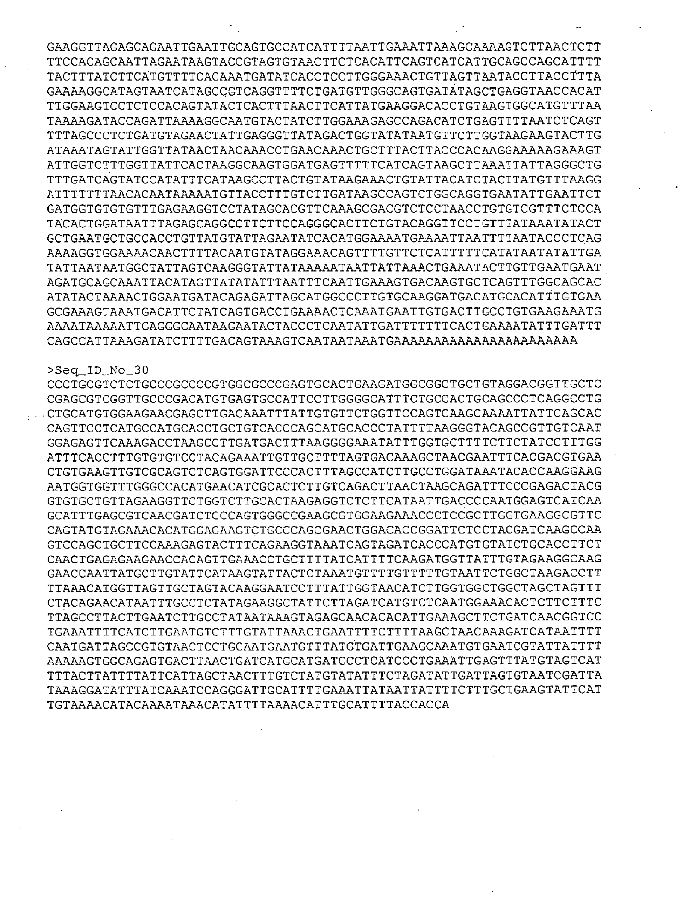

**Abbildung 4** zeigt die Nukleinsäuresequenzen der 30 Markergene, die in der vorliegenden Erfindung differentiell zwischen Patienten mit und ohne Progression des primär aufgetretenen kolorektalen Karzinoms exprimiert sind, wenn im Validations-Bootstrap in jeder Iteration zwei Datensätze als Testset verwandt werden.

### Patienten und Tumor Charakterisierung

Das Patientenkollektiv für die Entwicklung der Signatur bestand aus 55 Patienten, 34 Männer und 21 Frauen, bei denen ein kolorektales Karzinom diagnostiziert wurde. Diese Patienten wurden im Zeitraum August 1988 bis Juni 1998 operiert, um das kolorektale Karzinom total zu entfernen. Das Alter der Patienten zum Zeitpunkt der Operation reichte von 33 Jahre bis 87 Jahre, das mittlere Alter betrug 63,4 Jahre.

Unter den 55 entfernten Karzinomen wurden 11 als Tumore im UICC Stadium I (TNM-Klassifikation: pT1 oder pT2 und pN0 und pM0) und 44 als Tumore im UICC Stadium II klassifiziert (TNM - Klassifikation: pT3 oder pT4 und pN0 und pM0).

Die Gesamtbeobachtungszeit der Patienten, das ist die Zeit von der ersten durchgeführten Operation bis zur letzten Beobachtung eines Patienten, betrug im Mittel 11,25 Jahre, das Minimum war 6,36 Jahre, das Maximum war 16,53 Jahre.

Nach der Operation wurde bei 26 Patienten eine Progression der Erkrankung diagnostiziert, 29 Patienten blieben nach ihrer Operation progressiönsfrei.

### Beispiel 1: RNA Extraktion und Target Labeling

Die Tumoren wurden homogenisiert und die RNA wurde mittels des RNeasy Mini Kit (Qiagen, Hilden, Deutschland). isoliert und in 55µl Wasser aufgenommen. Die cRNA Präparation wurde wie bereits beschrieben (Birkenkamp-Demtroder K, Christensen LL, Olesen SH, et al. Gene expression in colorectal cancer. Cancer Res 2002; 62:4352-63) durchgeführt. Doppelsträngige cDNA wurde mittels eines Oligo-dT-T7 primer (Eurogentec, Koeln, Germany) synthetisiert und anschließend mit Hilfe des Promega RiboMax T7-kit (Promega, Madison, Wisconsin) und Biotin-NTP Markierungsmix (Loxo, Dossenheim, Germany) transkribiert.

Fünfzehn Mikrogramm cRNA wurden anschließend bei 95°C 35 Minuten lang fragmentiert.

### Beispiel 2: Mikroarray Experimente

Die cRNA wurde mit dem B2-Kontrolloligonukleotid (Affymetrix, Santa Clara, CA), eukaryotischen Hybridisierungskontrollen (Affymetrix, Santa Clara, CA), Heringssperm (Promega, Madison, Wisconsin), Hybridisierungspuffer and BSA auf ein finales Volumen von 300µl aufgefüllt und auf einen Mikroarraychip U133A (Affymetrix, Santa Clara, CA) 16 Stunden lang bei 45°C hybridisiert. Die Wasch- and Inkubationsschritte mit Streptavidin (Roche, Mannheim), biotinyliertem Ziege-Anti-Streptavidin Antikörper (Serva, Heidelberg), Ziege-IgG (Sigma, Taufkirchen) and Streptavidin-Phycoerythrin Konjugat (Molecular Probes, Leiden, Niederlande) wurde in einer Affymetrix Fluidics Station entsprechend dem Herstellerprotokoll durchgeführt.

Die Arrays wurden anschließend mit einem auf HP-Argon-Ionenlaser basierten konfokalen Mikroskop gescannt und die digitalisierten Bilddaten wurden mit Hilfe der Affymetrix® Microarray Suite 5.0 Software prozessiert. Die Genechips wurden dabei einer Qualitätskontrolle unterzogen um Scans mit abnormalen Charakteristika zu entfernen. Die Kriterien waren: zu hoher oder zu niedriger dynamischer Bereich, hohe Sättigung der "perfect matches", hohes Pixelrauschen, "grid misalignment" Probleme und ein niedriges mittleres Signal-zu-Rauschen Verhältnis.

### Beispiel: 3 Bioinformatische Auswertung

Die statistische Datenanalyse wurde mit der Open-Source Software R, Version 2.3 und den Bioconductor Paketen, Version 1.8 ausgeführt. Ausgehend von den 55 CEL-Files, die Ausgabe der oben genannten Affymetrix Software sind, wurden die Gen-Expressionswerte durch FARMS Kondensierung [Hochreiter et al. (2006), Bioinformatics 22(8):943-9] bestimmt.

Basierend auf den klinischen Daten von 55 Patienten wurde das Klassifikationsproblem "Klassifikation der 55 Expressionsdatensätze nach progressionsfreiem Überleben der zugehörigen Patienten" formuliert und analysiert. Das Expressionsdatenset stammte von den oben beschriebenen Patienten, von denen bei 26 eine Progression auftrat und bei 29 progressionsfreies Überleben dokumentiert wurde. Die in der Erfindung beschriebenen Markergene wurden, wie in Abbildung 3 gezeigt, mit einem zweifach geschachteltem Bootstrap-Ansatz [Efron (1979) Bootstrap Methods - Another Look at the Jackknifing, Ann. Statist. 7, 1-6], bestimmt. In der äußeren Schleife, dem sogenannten Validations-Bootstrap mit 500 Iterationen wurden die Daten zufällig partitioniert in ein Testset und ein Trainingset. Die Größen dieser Sets wurden folgendermaßen variiert:
a) Es wurde ein Datensatz in das Testset gewählt, 54 bilden das Trainingset.
b) Es wurden zwei Datensätze in das Testset gewählt, 53 bilden das Trainingset.
c) Es wurden drei Datensätze in das Testset gewählt, 52 bilden das Trainingset.

Anhand der Trainingsdaten wurde in der inneren Bootstrap-Schleife durch eine Random-Forest-Analyse die Featurerelevanz aus den Daten extrahiert. Dazu wurden in 50 inneren Schleifeniterationen jeweils 10 Datensätze als inneres Trainingset zufällig gezogen. Diese wurden durch eine SVM, die auf den 44, 43, oder 42 verbliebenen Datensätzen trainiert wurde klassifiziert und der Einfluss eines Features aus dessen Beitrag zum Klassifikationsfehler ermittelt: Stieg der Fehler durch Permutieren der Werte eines Features auf den 10 Testdatensätzen, während die Werte aller anderen Features unverändert blieben, so wurde dieses Feature stärker gewichtet. Anhand einer Häufigkeitstabelle wurden die 30 in den inneren Schleifeniterationen am häufigsten gewählten Features ermittelt und für die Prognose der zwei Testdatensätze der äußeren Schleife verwendet: eine Support-Vector-Machine mit linearem Kernel (Kostenparameter = 10) wurde auf den 54, 53 oder 52 Datensätzen des äußeren Trainingssets trainiert und dann auf die ein, zwei oder drei Testdatensätze angewandt. Nach 500 Iterationen wurden die durchschnittliche, prospektive Klassifikationsrate (mit Sensitivität und Spezifität) und die Häufigkeiten der gefundenen Features bestimmt.

Die Gensignaturen beinhalten nur Features, die mit großer Häufigkeit in allen Ziehungen relevant waren und wurden entsprechend der relativen Häufigkeit sortiert. In der retrospektiven "Leave-One-Out-Cross Validation" (LOOCV) der Signaturen wurden im Fall von sieben verwendeten Features 80% der Datensätze richtig klassifiziert. (siehe auch Tabelle 2a, 2b, und 2c)

Im Fall b), wo zwei Testsamples entnommen wurden, beinhaltet die resultierende Gensignatur elf Features, die in mehr als 50% aller Ziehung relevant waren. Diese wurden entsprechend der relativen Häufigkeit sortiert. In der retrospektiven Kreuzvalidierung (500 Leave-10-Out-CV) der 11er-Signatur wurden 86% der Datensätze richtig klassifiziert. Die durchschnittliche prospektive Klassifikationsrate wurde für diesen Fall mit 76% bestimmt.

**Tabelle 1a: Markergene die eine Vorhersage zwischen progressionsfreiem Überleben und Progression der Erkrankung nach der Entfernung des primären kolorektalen Karzinoms (PFS) ermöglichen. Die Frequenz stellt die Häufigkeit dar mit der das jeweilige Gen in den inneren Bootstrap-Schleifen einen großen Beitrag zum Klassifikationsergebnis lieferte (siehe auch Beschreibung im Beispiel 3). Hier wurde im Validations-Bootstrap in jeder Iteration ein Datensatz als Testset verwandt.**

| Sequenz ID | Affymetrix ID | HUGO ID | RefSeq Nummer | Frequenz |
|---|---|---|---|---|
| 1 | 210154_at | ME2 | NM_002396 | 1,00000 |
| 2 | 215719_x_at | FAS | NM_000043 | 1,00000 |
| 3 | 207088_s_at | SLC25A11 | NM_003562 | 1,00000 |
| 4 | AFFX-HUMISGF3A/M97935_MB_at | STAT1 | NM_007315 | 1,00000 |
| 6 | 202543_s_at | GMFB | NM_004124 | 1,00000 |
| 8 | 209397_at | ME2 | NM_002396 | 0,99917 |
| 7 | 204533_at | CXCL10 | NM_001565 | 0,99833 |
| 5 | 214464_at | CDC42BPA | NM_003607 | 0,99667 |
| 12 | 200628_s_at | WARS | NM_004184 | 0,99333 |
| 13 | 201695_s_at | NP | NM_000270 | 0,99083 |
| 10 | 212316_at | NUP210 | NM_024923 | 0,98833 |
| 9 | AFFX-HUMISGF3A/M97935_MA_at | STAT1 | NM_007315 | 0,98000 |
| 14 | 220892_s_at | PSAT1 | NM_021154 | 0,97833 |
| 17 | 201761_at | MTHFD2 | NM_006636 | 0,96083 |
| 11 | 212254_s_at | DST | NM_001723 | 0,95000 |
| 15 | 221481_x_at | HNRPD | NM_001003810 | 0,94167 |
| 16 | 209003_at | SLC25A11 | NM_003562 | 0,93167 |
| 19 | 207332_s_at | TFRC | NM_003234 | 0,92000 |
| 20 | 218096_at | AGPAT5 | NM_018361 | 0,87583 |
| 18 | 208969_at | NDUFA9 | NM_005002 | 0,83917 |
| 23 | 201435_s_at | EIF4E | NM_001968 | 0,83500 |
| 21 | 209832_s_at | CDT1 | NM_030928 | 0,82917 |
| 22 | 208691_at | TFRC | NM_003234 | 0,77750 |
| 26 | 200754_x_at | SFRS2 | NM_003016 | 0,72833 |
| 27 | 209892_at | FUT4 | NM_002033 | 0,64917 |
| 29 | 201730_s_at | TPR | NM_003292 | 0,63333 |
| 24 | 202336_s_at | PAM | NM_000919 | 0,61583 |
| 25 | 207029_at | KITLG | NM_000899 | 0,55083 |
| 30 | 201619_at | PRDX3 | NM_006793 | 0,48750 |
| 28 | 202589_at | TYMS | NM_001071 | 0,45333 |

**Tabelle 1b: Markergene die eine Vorhersage zwischen progressionsfreiem Überleben und Progression der Erkrankung nach der Entfernung des primären kolorektalen Karzinoms (PFS) ermöglichen. Die Frequenz stellt die Häufigkeit dar mit der das jeweilige Gen in den inneren Bootstrap-Schleifen einen großen Beitrag zum Klassifikationsergebnis lieferte (siehe auch Beschreibung im Beispiel 3). Hier wurden im Validations-Bootstrap in jeder Iteration zwei Datensätze als Testset verwandt.**

| Sequenz ID | Affymetrix ID | HUGOID | RefSeq Nummer | Frequenz |
|---|---|---|---|---|
| 1 | 210154_at | ME2 | NM_002396 | 0,97796 |
| 2 | 215719_x at | FAS | NM_000043 | 0,85304 |
| 3 | 207088_s_at | SLC25A11 | NM_003562 | 0,8286 |
| 4 | AFFX-HUMISGF 3A/M97935_MB_at | STAT1 | NM_007315 | 0,74488 |
| 5 | 214464_at | CDC42BPA | NM_003607 | 0,6098 |
| 6 | 202543_s_at | GMFB | NM_004124 | 0,58552 |
| 7 | 204533_at | CXCL10 | NM_001565 | 0,58524 |
| 8 | 209397_at | ME2 | NM_002396 | 0,56704 |
| 9 | AFFX-HUMISGF 3A/M97935_MA_at | STAT1 | NM_007315 | 0,5578 |
| 10 | 212316_at | NUP210 | NM_024923 | 0,51524 |
| 11 | 212254_s_at | DST | NM_001723 | 0,5 |
| 12 | 200628_s_at | WARS | NM_004184 | 0,48176 |
| 13 | 201695_s_at | NP | NM_000270 | 0,4772 |
| 14 | 220892_s_at | PSAT1 | NM_021154 | 0,47156 |
| 15 | 221481_x_at | HNRPD | NM_001003810 | 0,47156 |
| 16 | 209003_at | SLC25A11 | NM_003562 | 0,4464 |
| 17 | 201761_at | MTHFD2 | NM_006636 | 0,42148 |
| 18 | 208969_at | NDUFA9 | NM_005002 | 0,41196 |
| 19 | 207332_s_at | TFRC | NM_003234 | 0,40768 |
| 20 | 218096_at | AGPAT5 | NM_018361 | 0,40216 |
| 21 | 209832_s_at | CDT1 | NM_030928 | 0,39732 |
| 22 | 208691_at | TFRC | NM_003234 | 0,34728 |
| 23 | 201435_s_at | EIF4E | NM_001968 | 0,34504 |
| 24 | 202336_s_at | PAM | NM_000919 | 0,32592 |
| 25 | 207029_at | KITLG | NM_000899 | 0,32272 |
| 26 | 200754_x_at | SFRS2 | NM_003016 | 0,31884 |
| 27 | 209892_at | FUT4 | NM_002033 | 0,3174 |
| 28 | 202589_at | TYMS | NM_001071 | 0,27824 |
| 29 | 201730_s_at | TPR | NM_003292 | 0,27144 |
| 30 | 201619_at | PRDX3 | NM_006793 | 0,26516 |

**Tabelle 1c: Markergene die eine Vorhersage zwischen progressionsfreiem Überleben und Progression der Erkrankung nach der Entfernung des primären kolorektalen Karzinoms (PFS) ermöglichen. Die Frequenz stellt die Häufigkeit dar mit der das jeweilige Gen in den inneren Bootstrap-Schleifen einen großen Beitrag zum Klassifikationsergebnis lieferte (siehe auch Beschreibung im Beispiel 3). Hier wurden im Validations-Bootstrap in jeder Iteration drei Datensätze als Testset verwandt.**

| Sequenz ID | Affymetrix ID | HUGO ID | RefSeq Nummer | Frequenz |
|---|---|---|---|---|
| 1 | 210154_at | ME2 | NM_002396 | 1 |
| 3 | 207088_s_at | SLC25A11 | NM_003562 | 1 |
| 2 | 215719_x_at | FAS | NM_000043 | 1 |
| 4 | AFFX-HUMISGF3A/M97935_MB_at | STAT1 | NM_007315 | 0,997 |
| 6 | 202543_s_at | GMFB | NM_004124 | 0,991 |
| 7 | 204533_at | CXCL10 | NM_001565 | 0,962 |
| 8 | 209397_at | ME2 | NM_002396 | 0,9575 |
| 5 | 214464_at | CDC42BPA | NM_003607 | 0,9445 |
| 13 | 201695_s_at | NP | NM_000270 | 0,923 |
| 9 | AFFX-HUMISGF3A/M97935_MA_at | STAT1 | NM_007315 | 0,905 |
| 12 | 200628_s_at | WARS | NM_004184 | 0,891 |
| 10 | 212316_at | NUP210 | NM_024923 | 0,869 |
| 14 | 220892_s_at | PSAT1 | NM_021154 | 0,8565 |
| 17 | 201761_at | MTHFD2 | NM_006636 | 0,8295 |
| 11 | 212254_s_at | DST | NM_001723 | 0,8015 |
| 16 | 209003_at | SLC25A11 | NM_003562 | 0,7745 |
| 15 | 221481_x_at | HNRPD | NM_001003810 | 0,765 |
| 19 | 207332_s_at | TFRC | NM_003234 | 0,7415 |
| 23 | 201435_s_at | EIF4E | NM_001968 | 0,7005 |
| 21 | 209832_s_at | CDT1 | NM_030928 | 0,6875 |
| 20 | 218096_at | AGPAT5 | NM_018361 | 0,664 |
| 18 | 208969_at | NDUFA9 | NM_005002 | 0,661 |
| 26 | 200754_x_at | SFRS2 | NM_003016 | 0,612 |
| 22 | 208691_at | TFRC | NM_003234 | 0,601 |
| 27 | 209892_at | FUT4 | NM_002033 | 0,5715 |
| 25 | 207029_at | KITLG | NM_000899 | 0,503 |
| 24 | 202336_s_at | PAM | NM_000919 | 0,492 |
| 28 | 202589_at | TYMS | NM_001071 | 0,468 |
| 30 | 201619_at | PRDX3 | NM_006793 | 0,4455 |
| 29 | 201730_s_at | TPR | NM_003292 | 0,427 |

**Tabelle 2a: Sensitivität, Spezifität und korrekte Klassifikationsrate der Klassifikation des Auftretens einer Progression innerhalb von fünf Jahren nach der primären Diagnose eines kolorektalen Karzinoms in Abhängigkeit von der verwendeten Markergenanzahl. Die Menge der verwendeten Gene ist monoton wachsend, das heißt in Zeile 9 sind alle Gene von SEQ ID 1 bis SEQ ID 9 und in Zeile 6 alle Gene von SEQ_ID 1 bis SEQ_ID 6 zur Signaturbestimmung herangezogen (siehe auch Abbildung 2a). Hier wurde im Validations-Bootstrap in jeder Iteration ein Datensatz als Testset verwandt.**

| SEQ_ID | HUGO_ID | Sensitivität hohes Rezidivrisiko) | Spezifität (für niedriges Rezidivrisiko) | Klassifikationsrate |
|---|---|---|---|---|
| 1 | ME2 | NA | NA | NA |
| 2 | FAS | 0,88 | 0,72 | 0,80 |
| 3 | SLC25A11 | 0,85 | 0,76 | 0,80 |
| 4 | STAT1 | 0,77 | 0,83 | 0,80 |
| 6 | GMFB | 0,81 | 0,83 | 0,82 |
| 8 | ME2 | 0,81 | 0,83 | 0,82 |
| 7 | CXCL10 | 0,73 | 0,79 | 0,76 |
| 5 | CDC42BPA | 0,73 | 0,93 | 0,84 |
| 12 | WARS | 0,73 | 0,90 | 0,82 |
| 13 | NP | 0,73 | 0,90 | 0,82 |

**Tabelle 2b: Sensitivität, Spezifität und korrekte Klassifikationsrate der Klassifikation des Auftretens einer Progression innerhalb von fünf Jahren nach der primären Diagnose eines kolorektalen Karzinoms in Abhängigkeit von der verwendeten Markergenanzahl. Die Menge der verwendeten Gene ist monoton wachsend, das heißt in Zeile 9 sind alle Gene von SEQ_ID 1 bis SEQ ID 9 und in Zeile 6 alle Gene von SEQ_ID 1 bis SEQ_ID 6 zur Signaturbestimmung herangezogen (siehe auch Abbildung 2b). Hier wurden im Validations-Bootstrap in jeder Iteration zwei Datensätze als Testset verwandt.**

| SEQ_ID | HUGO_ID | Sensitivität (für hohes Rezidivrisiko) | Spezifität (für niedriges Rezidivrisiko) | Klassifikationsrate |
|---|---|---|---|---|
| 1 | ME2 | NA | NA | NA |
| 2 | FAS | 0,88 | 0,72 | 0,80 |
| 3 | SLC25A11 | 0,85 | 0,76 | 0,80 |
| 4 | STAT1 | 0,77 | 0,76 | 0,76 |
| 5 | CDC42BPA | 0,85 | 0,90 | 0,87 |
| 6 | GMFB | 0,81 | 0,86 | 0,84 |
| 7 | CXCL10 | 0,85 | 0,90 | 0,87 |
| 8 | ME2 | 0,85 | 0,90 | 0,87 |
| 9 | STAT1 | 0,88 | 0,90 | 0,89 |
| 10 | NUP210 | 0,81 | 0,90 | 0,85 |

**Tabelle 2c: Sensitivität, Spezifität und korrekte Klassifikationsrate der Klassifikation des Auftretens einer Progression innerhalb von fünf Jahren nach der primären Diagnose eines kolorektalen Karzinoms in Abhängigkeit von der verwendeten Markergenanzahl. Die Menge der verwendeten Gene ist monoton wachsend, das heißt in Zeile 9 sind alle Gene von SEQ ID 1 bis SEQ_ID 9 und in Zeile 6 alle Gene von SEQ_ID 1 bis SEQ_ID 6 zur Signaturbestimmung herangezogen (siehe auch Abbildung 2c). Hier wurden im Validations-Bootstrap in jeder Iteration drei Datensätze als Testset verwandt.**

| SEQ_ID | HUGO_ID | Sensitivität (für hohes Rezidivrisiko) | Spezifität (für niedriges Rezidivrisiko) | Klassifikationsate |
|---|---|---|---|---|
| 1 | ME2 | NA | NA | NA |
| 3 | SLC25A11 | 0,85 | 0,76 | 0,80 |
| 2 | FAS | 0,85 | 0,76 | 0,80 |
| 4 | STAT1 | 0,77 | 0,83 | 0,80 |
| 6 | GMFB | 0,81 | 0,83 | 0,82 |
| 7 | CXCL10 | 0,85 | 0,83 | 0,84 |
| 8 | ME2 | 0,73 | 0,79 | 0,76 |
| 5 | CDC42BPA | 0,73 | 0,93 | 0,84 |
| 13 | NP | 0,77 | 0,90 | 0,84 |
| 9 | STAT1 | 0,77 | 0,90 | 0,84 |

## Patentansprüche

1. Verfahren zur Vorhersage der Wahrscheinlichkeit der Progression eines kolorektalen Karzinoms in Patienten mit Darmkrebs, umfassend die Bestimmung eines Genexpressionsprofils der Markergene ME2 und FAS wie dargestellt in SEQ ID NOs: 1 und 2 oder von zwei Markergenen, die mindestens 90 % Identität zu einem der in SEQ ID NO: 1 und 2 dargestellten Markergene haben, wobei das Expressionsprofil der Markergene durch die Messung der Quantität an Markergen-mRNA bestimmt wird und wobei das Expressionsprofil der Markergene aus einer Tumorprobe des Patienten stammt.

2. Verfahren nach Anspruch 1, bei dem das Expressionsprofil der Markergene mit einem Referenzmuster verglichen wird, welches indikativ für ein Wiederauftreten des Kolonkarzinoms eines Patienten ist.

3. Verfahren nach Anspruch 2, wobei der Vergleich der Expressionsprofile mit einem Verfahren zur Mustererkennung durchgeführt wird.

4. Verfahren nach Anspruch 3, bei dem die Mustererkennungsmethode aus einem zweifach geschachtelten Bootstrap-Ansatz in Kombination mit einer Decision-Tree-Analyse oder aus einem zweifach geschachtelten Bootstrap-Ansatz in Kombination mit einer Random Forest-Analyse für Bestimmung der Einzelrelevanz der Gene besteht

5. Verfahren nach einem der Ansprüche 1 bis 4, bei dem ein primäres Kolonkarzinom, insbesondere der Stadien UICC-1 oder UICC-II, untersucht wird.

6. Verfahren nach Anspruch 1, wobei die Quantität von Markergen-RNA mittels Genchiptechnologie, (RT-) PCR, Northern Hybridisierung, Dot-Blotting oder in situ Hybridisierung bestimmt wird.

7. Verwendung eines cDNA- oder Oligonukleotid-Mikroarrays enthaltend Sequenzen gemäß SEQ ID NOs: 1 und 2 oder ihren revers-komplementären Sequenzen in einem Verfahren nach Anspruch 1 bis 6.

8. Verwendung eines Kits in einem Verfahren nach Anspruch 1 bis 6, wobei der Kit ein cDNA- oder Oligonukleotid-Mikroarray enthaltend Sequenzen gemäß SEQ ID NOs: 1 und 2 oder ihren revers-komplementären Sequenzen enthält.

## Claims

1. Method for the prediction of the likelihood of progression of a colorectal carcinoma in patients with colorectal cancer, comprising the determination of a gene expression profile of the marker genes ME2 and FAS as depicted in SEQ ID NOs 1 and 2 or of two marker genes which have an identity of at least 90 % to one of the marker genes depicted in SEQ ID NOs 1 and 2, wherein the expression profile of the marker genes is determined through the measurement of the quantity of marker gene mRNA and wherein the expression profile of the marker genes is derived from a tumor sample of the patient.

2. The method of claim 1, wherein the expression profile of the marker genes is compared to a reference pattern which is indicative for a recurrence of the colon carcinoma of a patient.

3. The method of claim 2, wherein the comparison of the expression profiles is performed with a method for pattern recognition.

4. The method of claim 3, wherein the pattern recognition method is a double nested bootstrap approach in combination with a decision-tree-analysis or a double nested bootstrap approach in combination with a Random Forest-analysis for the determination of the individual relevance of the genes.

5. The method of one of claims 1 to 4, wherein a primary colon carcinoma, in particular of stages UICC-I or UICC-II is analyzed.

6. The method of claim 1, wherein the quantity of marker gene RNA is determined using gene chip technology, (RT-) PCR, Northern Hybridization, Dot-Blotting or in situ hybridization.

7. Use of a cDNA- or oligo-nucleotide microarray comprising sequences according to SEQ ID NOs 1 and 2 or their revers-complementary sequences in a method of claims 1 to 6.

8. Use of a kit in a method of claims 1 to 6, wherein the kit comprises a cDNA- or oligo-nucleotide microarray comprising sequences according to SEQ ID NOs 1 or 2 or their revers-complementary sequences.

## Revendications

1. Procédé pour la prédiction de la probabilité de la progression d'un cancer colorectal chez des patients présentant un cancer de l'intestin, comprenant la détermination de l'expression génique des gènes marqueurs ME2 et FAS tels que représentés dans SEQ ID N° 1 et 2, ou de deux gènes marqueurs, qui ont une identité d'au moins 90 % avec l'un des gènes marqueurs représentés dans SEQ ID N° 1 et 2, le profil d'expression des gènes marqueurs étant déterminé par la mesure de la quantité d'ARNm des gènes marqueurs, et le profil d'expression des gènes marqueurs dérivant d'un échantillon de tumeur du patient.

2. Procédé selon la revendication 1, dans lequel le profil d'expression des gènes marqueurs est comparé à un modèle de référence, qui est indicatif d'une récurrence du cancer colorectal d'un patient.

3. Procédé selon la revendication 2, dans lequel la comparaison des profils d'expression est mise en oeuvre à l'aide d'un procédé de reconnaissance de modèles.

4. Procédé selon la revendication 3, dans lequel la méthode de reconnaissance des modèles est constituée d'une approche en rééchantillonnage (bootstrap) à double emboîtement en combinaison avec une analyse par arbre de décision, ou d'une approche en rééchantillonnage à double emboîtement en combinaison avec une analyse par forêt aléatoire pour déterminer la pertinence individuelle des gènes.

5. Procédé selon l'une des revendications 1 à 4, dans lequel c'est un cancer primaire du côlon, en particulier des stades UICC-I ou UICC-II, qui est examiné.

6. Procédé selon la revendication 1, dans lequel la quantité de l'ARN des gènes marqueurs est déterminée par la technologie des puces à ADN, la (RT)PCR, l'hybridation de Northern, le dot-blotting ou l'hybridation in situ.

7. Utilisation d'un microréseau d'ADNc ou d'oligonucléotides contenant des séquences selon SEQ ID N° 1 et 2 ou leurs séquences complémentaires inverses dans un procédé selon les revendications 1 à 6.

8. Utilisation d'une trousse dans un procédé selon les revendications 1 à 6, pour laquelle la trousse contient un microréseau d'ADNc ou d'oligonucléotides contenant des séquences selon SEQ ID N° 1 et 2 ou leurs séquences complémentaires inverses.
